# EUROPÄISCHE PATENTSCHRIFT

(11) **EP 3 784 686 B1**
(45) Veröffentlichungstag und Bekanntmachung des Hinweises auf die Patenterteilung: **07.06.2023**
(21) Anmeldenummer: 20710414.2
(22) Anmeldetag: 14.02.2020
(51) Int. Cl.: C07K 14/005, A61K 39/12, G01N 33/569

(54) **RUBELLA VIRUS SPIKE KONSTRUKT**
RUBELLA VIRUS SPIKE CONSTRUCT
STRUCTURE DE SPICULE DU VIRUS DE LA RUBÉOLE

(30) Priorität: 10.07.2019 DE 102019004812
(43) Veröffentlichungstag der Anmeldung: 03.03.2021
(73) Patentinhaber: Institut Virion-Serion GmbH Würzburg, 97076 Würzburg (DE); Stürmer, Judith, 97076 Würzburg (DE)
(72) Erfinder: SCHUMACHER, Thomas, 91056 Erlangen (DE); ARENDS, Hugo M., 97078 Würzburg (DE)
(74) Vertreter: Rudolph, Ulrike
(86) Internationale Anmeldenummer: PCT/DE2020/000024
(87) Internationale Veröffentlichungsnummer: WO 2021/004561

(56) Entgegenhaltungen:
- WO-A1-2017/060857
- EKATERINA K. PETROVA ET AL: "The key role of rubella virus glycoproteins in the formation of immune response, and perspectives on their use in the development of new recombinant vaccines", VACCINE, Bd. 34, Nr. 8, 1. Februar 2016 (2016-02-01), Seiten 1006-1011, XP055697416, AMSTERDAM, NL ISSN: 0264-410X, DOI: 10.1016/j.vaccine.2016.01.010
- PERRENOUD G ET AL: "A recombinant rubella virus E1 glycoprotein as a rubella vaccine candidate", VACCINE, ELSEVIER, AMSTERDAM, NL, Bd. 23, Nr. 4, 9. Dezember 2004 (2004-12-09), Seiten 480-488, XP004629183, ISSN: 0264-410X, DOI: 10.1016/J.VACCINE.2004.06.030 in der Anmeldung erwähnt
- SHAIMA NASR ELDEEN MOHAMED ELGENAID ET AL: "Prediction of Multiple Peptide Based Vaccine from E1, E2 and Capsid Proteins of Rubella Virus: An In-Silico Approach", IMMUNOME RESEARCH, Bd. 14, Nr. 1, 1. Januar 2018 (2018-01-01) , XP055695855, DOI: 10.4172/1745-7580.1000146

## Beschreibung

Die Erfindung betrifft ein Rubella Virus Spike Konstrukt, geeignet und vorgesehen für diagnostische und/oder therapeutische Anwendungen.

Das Rubella-Virus (auf Deutsch: Röteln-Virus) ist der Erreger der Röteln (Rubella, Rubeola). Röteln ist eine aerogene (d.h. sich über die Luft verbreitende) hochansteckende Infektionskrankheit, die eine lebenslange Immunität hinterlässt. Bei Kindern treten als typische Krankheitssymptome der Röteln rote Hautflecken (Exanthem), Fieber und eventuell Lymphknotenschwellungen auf. Komplikationen sind normalerweise selten. Eine Rötelninfektion während der Schwangerschaft kann jedoch zu schweren Komplikationen mit ausgeprägten Fehlbildungen des Kindes und zu Fehlgeburten führen. Wenn sich eine schwangere Frau während der ersten drei Schwangerschaftsmonate mit dem Rubella-Virus (RUBV) infiziert ist, besteht ein Risiko von 20%, dass der Fötus ein angeborenes Rubella-Syndrom (CRS) entwickelt. RUBV ist weltweit endemisch und eine Infizierung kann durch Impfungen verhindert werden (Hobman et al., 2007).

Das Rubella-Virus ist das einzige Mitglied der Gattung *Rubivirus* und gehört zur Familie der *Togaviridae*, deren Genom typischerweise aus einer einzelsträngigen RNA mit positiver Polarität besteht. Das RNA-Genom ist von einem ikosaedrischen Kapsid (T=4-Symmetrie) umgeben und hat eine Länge von etwa 10 kb (10.000 Nukleotiden). Es kodiert für drei strukturelle Proteine, nämlich das Kapsidprotein (31 kDa) und die beiden Hüllproteine E1 (58 kDa) und E2 (42-47 kDa), und für zwei nichtstrukturelle Proteine (p150 und p90) (Hobman et al., 2007). Das Kapsid ist von einer Lipidmembran (Virushülle) umgeben, die von der Wirtszellmembran abgeleitet ist. In dieser Lipidmembran sind die beiden Hüllproteine E1 und E2 in Form von Heterodimeren eingelagert sind. Beide Hüllproteine (E1 und E2) sind Glykoproteine und in der viralen Membran durch C-terminale Transmembrandomänen verankert (DuBois et al., 2012).

Die Rubella-Virionen (Synonym für: Rubella-Viruspartikel außerhalb einer Wirtszelle) haben eine Größe von 50 bis 70 nm im Durchmesser. Die Virionen bilden eine Vielzahl von Formen, die von nahezu kugelförmigen bis hin zu langgestreckten rohrförmigen Strukturen reichen (Battisti et al., 2012). Nach derzeitigem Wissensstand ist die Form der Oberflächenstruktur bei allen Rubella-Virionen gleich und es existiert somit nur ein einziger Serotyp.

Das Hüllprotein E1 ist für die Erkennung und Bindung der Rubella-Virionen an zelluläre Rezeptoren (der Wirtszellen) verantwortlich, und es ist an der Membranfusion beteiligt (DuBois et al., 2012). Das Hüllprotein E2 wird für die effiziente Faltung und den Transport von E1 durch die betreffenden Kompartimente der Wirtszelle benötigt. Von den beiden Hüllproteinen ist nur die Struktur der E1-Ektodomäne in ihrer trimerischen, post-fusionären Konformation bekannt (Prasad et al., 2017).

Beide Hüllproteine E1 und E2 sind Transmembranglykoproteine vom Typ I. An der Oberfläche des Virions (Virion = Viruspartikel außerhalb der Wirtszelle) liegen sie als heterodimerer Rubella-Virus E1- und E2-Glycoprotein Komplex in Form der sogenannten Spikes (Synonyme: Rubella-Spikes; E1-E2-Proteinkomplex; E1-E2-Heterodimer) vor. Während der Biogenese des Rubella-Virus in der Wirtszelle wird zunächst im Zuge der Translation ein Struktur-Polyprotein synthetisiert, das von Signalpeptidasen in das Capsidprotein und die beiden Hüllproteine E1 und E2 gespalten wird. Die Dimerisierung von E1 und E2 findet im endoplasmatischen Retikulum statt. Dabei fördert E2 als Chaperon die Faltung und damit die strukturelle Integrität von E1. Im Unterschied zu anderen *Togavidirae* durchlaufen E1 und E2 des Rubella-Virus keinen proteolytischen Reifungsschritt. Im Golgi-Apparat entstehen die Rubella-Viruspartikel, die den üblichen Sekretionsweg durchlaufen und schließlich in das extrazelluläre Milieu freigesetzt werden. (Dubé et al. 2014.)

Die Hauptfunktionen dieser Spikes (Synonym: Rubella-Spikes) sind die Bindung an/mit Rezeptoren der Wirtszelle und die Vermittlung der Fusion mit (Wirts-)Zellmembranen (Katow et al., 1988).

Im Stand der Technik gilt seit langem, dass das Hüllproteine E1 die wichtigste antigene Determinante ist, gegen die neutralisierende Antikörper des Wirtsorganismus gerichtet sind (Waxham et al., 1985). Aber auch das Hüllprotein E2 und das Capsid-Protein C gehören zu den Zielen der humoralen Immunantwort auf eine Rubella-Infektion. In Seren von mit Rubella infizierten Individuen (Patienten) sind Antikörper gegen E1 und E2 regelmäßig reichlich vorhanden.

Ein Vergleich (von Nedelkovic et al. 1999) der diagnostischen Potenziale von (in Sf9 Insektenzellen mit dem Baculovirus-System) rekombinant hergestellten Rubella-Proteinen C, E1 und E2 im Immunoblot- und Enzymimmunoassay (EIA) zeigte, dass die rekombinanten Proteine E1 und C vorherrschend die Immunantwort auslösten, sowohl bei postnatalen als auch bei vakzinalen Rubella-Virusinfektionen. Die Immunantwort gegen das rekombinante E2-Protein fiel wesentlich schwächer aus, im Fall einer angeborenen Infektion allerdings deutlich stärker.

Im Stand der Technik sind mehrere Ansätze bekannt, das Rubella-El-Hüllprotein für diagnostische Zwecke herzustellen. Viele Bemühungen waren darauf gerichtet, stabile und lösliche Fragmente von E1 mit einer hohen Antigenität und in großen Mengen herzustellen. Eine Methode dafür war die Herstellung von stabil infizierten/transfizierten Zelllinien, die E1 und/oder E2 rekombinant exprimieren. Beispielsweise beschreiben bereits Seppänen et al. (1991) die Expression von Rubella-Glykoproteinen E1 und E2 in Spodoptera frugiperda Sf9 Insektenzellen unter Verwendung des Baculovirus-Expressionssystems.

In der EP1780282A1 ist die rekombinante Expression und Herstellung eines löslichen modifizierten Rubella-E1-Hüllproteins beschrieben, dem mindestens die C-terminale Transmembranregion, das Ankersegment und die Aminosäure 143 bis 164 im mittleren Teil des Proteinmoleküls fehlen. Dieses modifizierte E1-Hüllprotein enthält wenigstens denjenigen Bereich der Rubella-E1-Aminsosäuresequenz, der die Disulfidbrücken Cys 349 - Cys 352 und Cys 368 - Cys 401 umspannt und wenigstens die Aminosäuren 315-412 umfasst, und es ist mit einem FKBP-Chaperon fusioniert. Gemäß der Lehre der EP1782082A1 ist es wesentlich, dass beide Disulfidbrücken im C-terminalen Teil intakt, d.h. geschlossen sind, um eine Rubella-El-Variante zu erhalten, die ausreichend antigen und für den Nachweis von Antikörpern geeignet ist.

Die EP 2222694A1 offenbart die Herstellung weiterer rekombinant exprimierter löslicher Rubella-E1-Hüllantigene, denen im Vergleich zum nativen E1-Hüllprotein die Transmembranregion und das C-terminalen Ankersegment sowie das Segment mit den Aminosäuren 143 bis 164 im mittleren Teil des Moleküls fehlt, und die wenigstens zwei Disulfidbindungen enthalten, von die eine zwischen Cys 225 und Cys 235 (C13-C14) und die andere zwischen Cys 349 und Cys 352 (C17-C18) ausgebildet ist.

Orellana et al. 1999 beschreiben die Generierung sogenannte "Rubella-Virus mimicking Proteoliposomen", nämlich Liposomen, die (in Sf9 Insektenzellen mit dem Baculovirus-System) rekombinant hergestellte Rubella-Virus Hüllproteine E1 und E2 exponiert tragen. Diese Rubella-Virus mimicking Proteoliposomen wurden von Antikörpern mit Spezifität für die Rubella-Virus-Proteine E1 und E2 erkannt.

Die bekannten rekombinant erzeugten und nicht weiter assemblierten Rubella-Antigene, nämlich Glykoprotein E1, Glykoprotein E2 und Capsid Protein haben den Nachteil, dass ihre Reaktivität in Immunoassays und/oder ihre Herstellung beschränkt ist. In der akuten Phase einer Rubella-Virusinfektion produziert der Patientenorganismus vor allem IgM-Antikörper gegen Glykoprotein E1 und E2, die dem Immunsystem in Form der Heterodimere bzw. Spikes präsentiert werden. Wenn Glykoprotein E1 oder E2 gemäß dem Stand der Technik jeweils separat mit Hilfe rekombinanter Proteinexpressionssysteme hergestellt werden, entstehen jedoch keine Heterodimere. Auch ein nachträgliches Zusammenfügen von aufgereinigtem Glykoprotein E1 und E2 führt nicht zu einer naturgetreuen Assemblierung von heterodimeren Spikes.

Im Stand der Technik sind auch seit langem sogenannte "Virus-Like Particles" (VLPs) bekannt, auch von Rubella-Virus (RLPs - Rubella-Like Particles). In verschiedenen Studien wurde nachgewiesen, dass die Expression der drei Rubella-Strukturproteine C, E1, und E2 in stabil transfizierten CHO- oder BHK-Zelllinien zur Produktion von Rubella-VLPs führt, deren Größe, Morphologie und Dichte jeweils mit der des nativen Rubella-Virus übereinstimmte, die aber nicht-infektiös waren. Mit diesen Rubella-VLPs wurden in diagnostischen Immunoassays menschliche Anti-Rubella-Virus Antikörper nachgewiesen. Außerdem konnte mit diesen Rubella-VLPs bei Mäusen eine Immunisierung erreicht werden, nämlich die Bildung von spezifischen Antikörpern gegen Rubella-Virus-Strukturproteine und von virusneutralisierenden und hämagglutinationshemmenden Antikörpern (vgl. Review von Petrova et al. 2016).

Für die rekombinante Produktion von Rubella-VLPs werden generell alle strukturellen Proteine, Glykoprotein E1, Glykoprotein E2 und das Capsid-Protein, exprimiert. Die VLPs, die hierbei gebildet werden, bestehen aus dem Capsid, das von einer Lipiddoppelschicht umgeben ist, in der die viralen Glykoproteinen E1 und E2 eingebettet sind. Die VLPs sind damit den nativen Rubella-Viren strukturell sehr ähnlich. Ein Nachteil der Anwendung von Rubella-VLPs in Immunoassays ist das Vorhandensein des Capsid-Proteins, das im Verdacht steht, Kreuzreaktionen zu verursachen (ygl. US6670117B2). Vor allem IgM Immunoassays sind sehr anfällig für Kreuzreaktivität. Außerdem ist die Herstellung von Rubella-VLPs mit mehreren Nachteilen verbunden: Die Expressionsrate in den benötigten eukaryotischen Expressionssystemen ist in der Regel sehr niedrig. Die VLPs müssen aufwendig aus Zelllysaten oder aus Zellkulturüberständen aufgereinigt werden, womit oft erhebliche Ausbeuteverluste einhergehen. Die VLP-Präparate enthalten mehr Verunreinigungen als rekombinante Antigene (die mit Hilfe eines Affinitätstags aufgereinigt wurden), wodurch die Gefahr von unspezifischen Reaktionen erhöht ist.

Impfstoffe (Vakzinen) gehen Rubella-Virus basieren im Stand der Technik überwiegend auf attenuierten Rubella-Virus-Stämmen. Diese haben jedoch gravierende Nachteile: Sie können nicht bei Schwangeren oder Menschen mit Immunschwäche eingesetzt werden. Oft treten Nebenwirkungen wie Arthritis, Diabetes, Schäden am zentralen Nervensystem sowie allergische und anaphylaktische Reaktionen auf. Herstellung, Transport und Lagerung sind aufwendig. (Hobman et al 1994, Qiu et al. 1994 - Review von Petrova et al. 2016).

Ein erfolgversprechender Ersatz für die Lebend-Vakzine wird derzeit in einem rekombinanten Rubella-Hüllprotein E1 gesehen. (Perrenoud G. et al. 2004 - Review von Petrova et al. 2016).

Nach wie vor besteht der Bedarf nach Rubella-Antigen-Präparaten, die möglichst viele verschiedene Epitope des nativen infektiösen Rubella-Virus präsentieren und deshalb dazu geeignet sind, in diagnostischen Systemen, z. B. ELISA- oder CLIA-Systemen, verschiedenste Anti-Rubella-Antiköper aus humanen Patientenproben zu binden und damit zu detektieren und/oder als Vakzine-Wirkstoff die Bildung von Antikörpern im Patientenorganismus auszulösen.

Der vorliegenden Erfindung liegt die Aufgabe zugrunde, neue Rubella-Antigene bereitzustellen, die hochlöslich und immunologisch hochreaktiv (d.h. hoch antigen) sind, und die für den zuverlässigen Nachweis einer Rötelninfektion, nämlich einer Infektion mit dem Erreger Rubella, und/oder als Vakzine-Wirkstoff gut geeignet sind.

Eine Lösung dieser Aufgabe besteht in der Angabe eines Rubella-Virus E1-E2-Hüllprotein-Komplex-Konstruktes, kurz "Rubella-Spike-Konstrukt", das dadurch gekennzeichnet ist,
- dass es wenigstens eine E1-Komponente und eine E2-Komponente umfasst, die (entweder unmittelbar bzw. direkt. oder mittelbar) miteinander verbunden (bzw. gekoppelt) sind,
- dass die E1-Komponente aus dem E1-Hüllprotein besteht, dessen C-terminale Transmembranregion und intravirionale Domäne entfernt sind, und dessen N-Terminus die Ektodomäne des E1-Hüllproteins umfasst,
- dass die E2-Komponente aus dem E2-Hüllprotein besteht, dessen Transmembranregionen und intravirionale Domäne entfernt sind, und dessen N-Terminus die Ektodomäne des E2-Hüllproteins umfasst,
- und dass der C-Terminus der E2-Komponente (d.h. der C-Terminus der Ektodomäne des E2-Hüllproteins) mit dem N-Terminus der E1-Komponente (d.h. mit dem N-Terminus der Ektodomäne des E1-Hüllproteins) unmittelbar (in direkter Fusion) oder mittelbar, nämlich mittels bzw. über einen Linker, verbunden (gekoppelt) ist, so dass ein E1-E2-Fusionsprotein vorliegt.

Überraschenderweise wurde gefunden, dass solche erfindungsgemäßen Rubella-Spike-Konstrukte, d.h. E1-E2-Fusionsproteine nach der Expression in der Wirtszelle, erfolgreich im ER (Endoplasmatisches Retikulum) und Golgi-Apparat prozessiert und in löslicher Form sekretiert (sezerniert, abgesondert) werden, und dass sie offensichtlich einem nativen Heterodimer immunologisch so ähnlich sind, dass sie wie dieses von humanen Anti-Rubella-Virus-Antikörpern (z.B. aus Patientenseren) in bekannten diagnostischen Testsystemen erkannt und gebunden werden.

Vorzugsweise ist das erfindungsgemäße E1-E2-Fusionsprotein des Rubella-Spike-Konstrukts noch mit einer Signalsequenz (Synonyme: Signalpeptide, Transitpeptide) fusioniert (gekoppelt).

Signalsequenzen sind relative kurze (etwa 20-30 Aminosäuren umfassende) Aminosäuresequenzen, die am N-Terminus von Proteinen lokalisiert sind und Informationen für die Sekretion dieses Proteins enthalten, insbesondere für dessen Transportweg und eventuell auch dessen Faltung. Sie kommen ubiquitär in Prokaryoten und Eukaryoten vor und bestehen prinzipiell aus drei Abschnitten: einem positiv geladenen amino-terminalen Abschnitt (N-Region), einem mittleren hydrophoben Abschnitt (H-Region) und einem leicht polaren Carboxy-Abschnitt (C-Region). Aus zahlreichen unterschiedlichsten Studien zur Herstellung rekombinanter Proteine in Zelllinien ist bekannt, dass die Sekretionseffizienz und damit die Produktionsrate des rekombinanten Proteins durch den Einsatz von Signalsequenzen signifikant gesteigert werden kann.

Vorzugsweise ist das erfindungsgemäße E1-E2-Fusionsprotein mit einem Affinitäts-Tag versehen (gekoppelt), um insbesondere bei rekombinanter Herstellung eine effektive Aufreinigung zu ermöglichen. Als Affinitäts-Tag kommt insbesondere ein Streptavidin-Affinitätstag (Strep-Tag, Twin-Strep-Tag) oder ein Polyhistidin-Tag (His-Tag) in Betracht. Erfindungsgemäß ist der Affinitäts-Tag vorzugsweise C-terminal angekoppelt, eine Anordnung (Positionierung) am N-terminus oder im Linker kommt ebenfalls in Betracht.

Vorzugsweise umfasst die Ektodomäne des E1-Hüllproteins die Aminosäuren (der Positionen) 1-446 gemäß SEQ ID NO:6 oder (dem entsprechend) die Aminosäuren AA583-1028 in der Referenzsequenz UniProtKB/SwissProt - P08563 (POLS_RUBVM), Sequenz Update May 30, 2006 (Version 2 der Sequenz).

Vorzugsweise umfasst die Ektodomäne des E2-Hüllproteins die Aminosäuren (der Positionen) 1-234 gemäß SEQ ID NO:8 oder (dem entsprechend) die Aminosäuren AA301-534 in der Referenzsequenz UniProtKB/SwissProt - P08563 (POLS_RUBVM), Sequenz Update May 30, 2006 (Version 2 der Sequenz).

Falls ein Linker eingesetzt wird, ist dieser vorzugsweise ein flexibler Linker, der weiter vorzugsweise ganz oder überwiegend aus Glycin und Serin besteht, und der insbesondere als Kurz-Linker oder Lang-Linker ausgebildet sein kann.

Die Größe bzw. Länge des Linkers kann einen Einfluss auf die antigenen Eigenschaften der Rubella-Spikes haben. In der Praxis wurde beobachtet, dass - in Abhängigkeit von der eingesetzten diagnostischen Plattform und insbesondere bei Verwendung von Glycin-Serin-Linkern- Spike-Konstrukte mit einem 24 aa-Linker oftmals bessere antigene Eigenschaften zeigen als Spike-Konstrukte mit einem 8 aa-Linker.

Durch Modifikationen des Linkers, insbesondere Wahl seiner Größe bzw. Länge und/oder Aminosäurezusammensetzung, kann das Rubella-Antigen für bestimmte diagnostische oder therapeutische Anwendungen optimiert und maßgeschneidert ("tailor-made-antigens") werden.

Die Erzeugung (Herstellung/Produktion) des erfindungsgemäßen Rubella-Spike-Konstrukts erfolgt vorzugsweise in eukaryontischen Zellkulturen mit Hilfe von Expressionsvektoren. Hierfür wird mit denjenigen RNA-Sequenzen, die für die E1- und E2-Hüllproteinkomponenten im Spike-Konstrukt kodieren, und gegebenenfalls mit der Nukleotidsequenz, die für die gewählte Linker-Sequenz kodiert, eine rekombinante Nukleotidsequenz erstellt, die für das erfindungsgemäße Rubella-E1-E2-Fusionsprotein kodiert, und diese wird in einen Expressionsvektor in operativer Verknüpfung insertiert. Die Erfindung betrifft deshalb auch derartige rekombinante Nukleotidsequenzen (Synonym: rekombinante DNA-Moleküle) und vorzugsweise solche, die die Nukleotidsequenzen gemäß SEQ ID NO:5 und SEQ ID NO:7 umfassen.

Besonders bevorzugte rekombinante Nukleotidsequenzen bzw. DNA-Moleküle, die sich in der Praxis bereits gut bewährt haben, sind dadurch gekennzeichnet, dass sie die Nukleotidsequenz gemäß SEQ ID NO: 1 und/oder die Nukleotidsequenz gemäß SEQ ID NO: 3 umfassen, wobei die Signalsequenz am N-terminalen Ende fakultativ vorhanden ist, d.h. sie kann fehlen oder durch eine ähnlich funktionierende und wirkende ersetzt sein.

Bevorzugte E1-E2-Fusionsproteine bzw. Rubella-Spike-Konstrukte sind dadurch gekennzeichnet, dass sie die Aminosäuresequenz gemäß SEQ ID NO: 2 oder gemäß SEQ ID NO: 4 umfassen, wobei die Signalsequenz am N-terminalen Ende und/oder die Linker-Sequenz im mittleren Bereich dieser Aminosäuresequenzen fakultativ vorhanden ist, d.h. sie kann fehlen oder durch eine ähnlich funktionierende und wirkende ersetzt sein.

Die Erfindung betrifft deshalb zudem einen Expressionsvektor, der in operativer Verknüpfung eine Nukleotidsequenz umfasst, die für die Rubella-E1- und -E2-Hüllproteinkomponenten im Spike-Konstrukt - vereinfacht: für das Rubella-E1-E2-Fusionsprotein - kodiert.

Der Expressionsvektor ist vorzugsweise ein Transfervektor gemäß EP2307543B1; beispielsweise der Transfervektor "pExpres2.1", im Handel erhältlich bei Expres2ion Biotechnologies, Horsholm, Dänemark.

Die Erfindung betrifft auch eine Wirtszelle, die mit einem Expressionsvektor transformiert ist, der in operativer Verknüpfung eine Nukleotidsequenz umfasst, die für die Rubella-E1- und -E2-Hüllproteinkomponenten im Spike-Konstrukt - vereinfacht: für das Rubella-E1-E2-Fusionsprotein - kodiert.

Als Wirtszelle wird vorzugsweise eine Drosophila Schneider 2 (S2) Zelle (vgl. Schneider 1972) eingesetzt.

Die Drosophila S2-Zelllinie ist im Handel erhältlich, bei der DSMZ (Braunschweig, Deutschland) unter der Depotnummer DSMZ ACC 130 und bei der ATCC (American Type Culture Collection, Postfach 1549, Manassas, Virginia, USA) unter der Depotnummer CRL-1963 hinterlegt und der Öffentlichkeit uneingeschränkt zugänglich.

Es wurde gefunden, dass die erfindungsgemäßen Rubella-Spike-Konstrukte in Drosophila S2-Zellen derart exprimiert und sezerniert werden, dass sie die antigenen Eigenschaften der nativen heterodimeren Rubella-E2/E1-Spikes perfekt nachahmen. d.h. nahezu identisch oder jedenfalls derart abbilden, wie sie das native Virus in diagnostischen Tests zur Detektion von IgM und IgG Antikörpern tut.

Prinzipiell ist die Herstellung von Rubella-Spike-Konstrukten mittels Rekombinationstechniken nicht auf das Expressionssystem von stabil (dauerhaft) oder transient (vorübergehend) transfizierten Drosophila S2-Zellen beschränkt, sondern kann auch mittels anderer eukaryontischer Expressionssysteme wie z.B. dem Baculovirus-Expressionssystem erfolgen.

Bei Verwendung von stabil (dauerhaft) oder transient (vorübergehend) transfizierten Drosophila S2-Zellen als Expressionssystem ist die im Rubella-Spike-Konstrukt vorzugsweise vorgesehene Signalsequenz vorzugsweise die Drosophila-BiP Signalsequenz. Diese hat sich in der Praxis als besonders gut geeignet erwiesen. Andere funktionsanaloge Drosophila-Signalsequenzen kommen ebenfalls in Betracht.

Bei Verwendung von anderen Expressionssystemen können andere Signalsequenzen erforderlich und/oder vorteilhafter sein, z.B. die gp64- oder HMS-Signalsequenz im Fall des Baculovirus-Expressionssystems.

Die Erfindung betrifft auch ein Verfahren zur Herstellung eines löslichen und immunreaktiven Rubella-Spike-Konstrukts, wobei das Verfahren die folgenden Schritte umfasst:
(a) Kultivierung von Wirtszellen, vorzugsweise Drosophila Schneider 2 (S2) Zellen;
(b) Transformieren der Wirtszellen mit einem Expressionsvektor, der eine für das Rubella-E 1-E2-Fusionsprotein kodierende Nukleotidsequenz in operativer Verknüpfung umfasst;
(c) Kultivierung der transfizierten Wirtszellen, wobei diese das Rubella-E 1 -E2-Fusionsprotein (vorzugsweise kontinuierlich) exprimieren und das Rubella-Spike-Konstrukte aus der Wirtszelle sekretieren/sezerniere;
(d) Aufreinigung des Fusionsproteins.

Die Erfindung betrifft außerdem ein Verfahren zum Nachweis und/oder zur Bestimmung und/oder zur Quantifizierung von Anti-Rubella-Antikörpern (insbesondere der Unterklassen IgG oder IgM oder beider) in einer menschlichen Probe, wobei das Rubella-Spike-Konstrukt als Fängerreagenz oder Bindungspartner oder beides für die Anti-Rubella-Antikörper verwendet wird.

Als Proben sind prinzipiell alle dem Fachmann bekannten biologischen Flüssigkeiten geeignet.

Die Erfindung umfasst ferner einen Reagenzkit (Testkit) zur Durchführung dieses Verfahrens zum Nachweis von Anti-Rubella-Antikörpern, wobei dieser Kit mindestens ein Rubella-Spike-Konstrukt als Antigen enthält.

Das Rubella-Spike-Konstrukt als Antigen kann insbesondere bei Einsatz in einem sogenannten "µ-capture ELISA" als spezifisches Konjugat für den selektiven Nachweise von gebundenen anti-Rubella-IgM-Antikörpern genutzt werden. Hierfür kann das Rubella-Spike-Konstrukt mit verschiedenen Tags (z. B. His6-Tag, (Twin)-Strep-Tag oder anderen Tags) versehen werden und dadurch das Antigen gezielt zur Herstellung von Konjugaten (enzymatisches Label, Fluorophore, etc.) optimal ausgelegt werden. Die Wirtschaftlichkeit der Konjugatherstellung wird damit entscheidend verbessert. Häufig genutzte, unselektive, chemischen Konjugationsverfahren, wie z. B. die Vernetzung von Proteinen mit Glutardialdehyd, können durch eine solche intrinsische gerichtet Kopplung mit hoher Effizienz und Reproduzierbarkeit ersetzt werden.

Das erfindungsgemäße Rubella-Spike-Konstrukt ist hochlöslich und immunologisch hochreaktiv (Synonym: hoch antigen), d.h. es ist sehr gut fähig, an für Rubella spezifische Antikörper zu binden oder von diesen erkannt und gebunden zu werden (z.B. im Fall von Anti-Rubella-El-Antikörpern in einer Probe). Es ist deshalb als Antigen für diagnostische Anwendungen sehr gut geeignet. Es bietet einen vollwertigen Ersatz für die Antigene aus Vollvirus, die nach dem Stand der Technik in unterschiedlichen diagnostischen Plattformen (ELISA, Partikel, etc.) eingesetzt werden.

Mit ihm gehen vor allem die Vorteile einher, dass bei der Herstellung keine Biostoffgefährdung durch potentiell infektiöses Material besteht, dass die Produktion unter S1 Bedingungen gemäß GenTG erfolgt, und dass durch Monoklonalisierung der Drosophila-S2-Zellen als Expressionssystem eine gesteigerte Ausbeute und verbesserte Standardisierbarkeit bei der Herstellung im Vergleich zum Stand der Technik erreicht wird. Weitere wichtige Vorteile sind eine kostengünstige Produktion durch Nutzung moderner biotechnologischer Verfahren wie z. B. Bioreaktoren (Batch oder Perfusion) und die Produktion in serumfreien Medien d.h. unter Eliminierung von potentiell infektiösen Proteinen (z.B. pathologisch veränderten Prionen wie im Fall von BSE) oder immunogenen Proteinen aus eingesetzten tierischen Komponenten wie insbesondere FCS oder BSA. Beide Vorteile bieten Zukunftssicherheit bei der Herstellung und Anwendung des Antigens sowohl unter ethischen Aspekte als auch regulatorischen Aspekten.

Aufgrund der prinzipiell uneingeschränkten Manipulierbarkeit der Komponenten des Rubella-Spike-Konstrukts können Anpassungen an neue technologische Anforderungen relativ einfach vorgenommen werden. Beispielsweise können Tags aller Art zur gerichteten Kopplung auf unterschiedlichste Oberflächen eingeführt werden, und die Herstellung von Konjugaten für µ-capture Tests auf verschiedenen Plattformen (ELISA, CLIA, fluoreszenzmarkierte Konjugate) ist ebenfalls ohne größere technische Schwierigkeiten realisierbar.

Da das erfindungsgemäße Rubella-Spike-Konstrukt die nativen heterodimeren Rubella-E2/E1-Spikes praktisch genauso abbildet wie das native Virus in diagnostischen Tests, ist es grundsätzlich zur Detektion jeder Art von Anti-Rubella-Antiköpern (insbesondere IgM, IgG und IgA) in diagnostischen (insbesondere serologischen) Tests geeignet.

Untersuchungen mittels immundiagnostischer Tests zur immunologischen Reaktivität der Rubella-Spike-Konstrukte als Antigene im Vergleich zu Rubella-Antigenen aus dem Stand der Technik zeigen, dass die erfindungsgemäßen Rubella-Spike-Konstrukt Antigene für den plattformübergreifend Einsatz in diagnostischen Testsystemen für die Rubella-IgM- und -IgG-Serologie gut geeignet sind. Hinsichtlich des im Stand der Technik aktuell eingesetzten Standardantigens, nämlich des isolierten Rubella-Vollvirus, bedeutet das eine deutliche Verbesserung, weil die erfindungsgemäßen Rubella-Spike-Konstrukte nahezu beliebig verändert ("tailor-made-antigens") und an bestehende und zukünftige diagnostische Verfahren und Anforderungen in der Rubella-Serologie angepasst werden können. Zudem ermöglichen die erfindungsgemäßen Rubella-Spike-Konstrukt-Antigene über die biotechnologische Produktion als rekombinante Proteine eine kosteneffektivere Herstellung und eine deutlich verbesserte Produkt- und Qualitätskonstanz im Vergleich zum bisherigen Standardantigen Vollvirus. Zusätzlich ist davon auszugehen, dass die prinzipiell nahezu uneingeschränkte Manipulierbarkeit des Rubella-Spike-Konstrukt-Proteins (z.B. Größe der Epitope, Zusammensetzung der Epitope, etc.) eine gezielte Beseitigung von störenden Einflüssen wie Kreuzreaktivität oder unspezifischen Reaktionen ermöglicht, und das plattformübergreifend, individuell für jede serologische Applikation in der Rubella-Serologie.

Aufgrund der verbesserten Qualität und Quantität zugänglicher nativ-artiger Konformationsepitope ist das Rubella-Spike-Konstrukt auch ein erfolgversprechender Kandidat für einen breit einsetzbaren Vakzine-Wirkstoff gegen Rubella-Infektionen.

Die Erfindung betrifft auch die Verwendung des Rubella-Spike-Konstrukts als Impfstoff. Gegenstand der Erfindung ist somit auch eine Vakzinezubereitung, die das Rubella-Spike-Konstrukt als antigenen Wirkstoff umfasst.

Die Herstellung von Impfstoffen, die ein immunogenes Polypeptid als Wirkstoff enthalten, ist dem Fachmann bekannt. Solche Impfstoffe werden üblicherweise als injizierbare Präparate (flüssige Lösungen oder Suspensionen) bereitgestellt. Der Wirkstoff, hier das Rubella-Spike-Konstrukt, wird mit Hilfsstoffen gemischt, die pharmazeutisch verträglich und mit dem Wirkstoff kompatibel sind (z. B. Wasser, wässerige physiologische Puffer, Salzlösung, Dextrose, Glycerin, Ethanol.)

Die Erfindung wird im Folgenden anhand von Ausführungsbeispielen und Abbildungen näher erläutert.

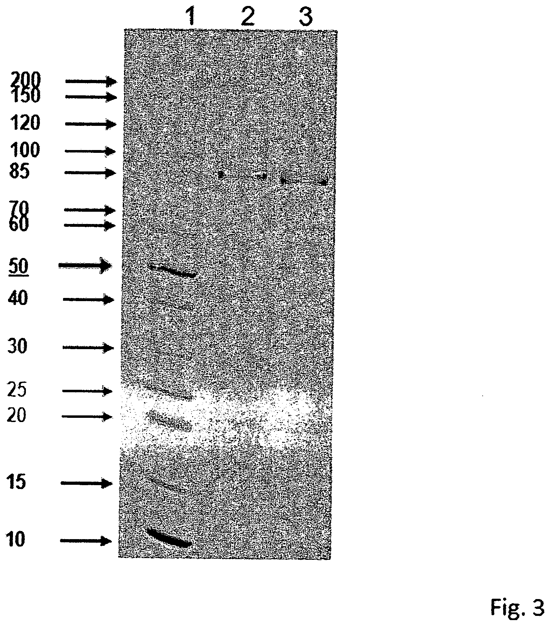

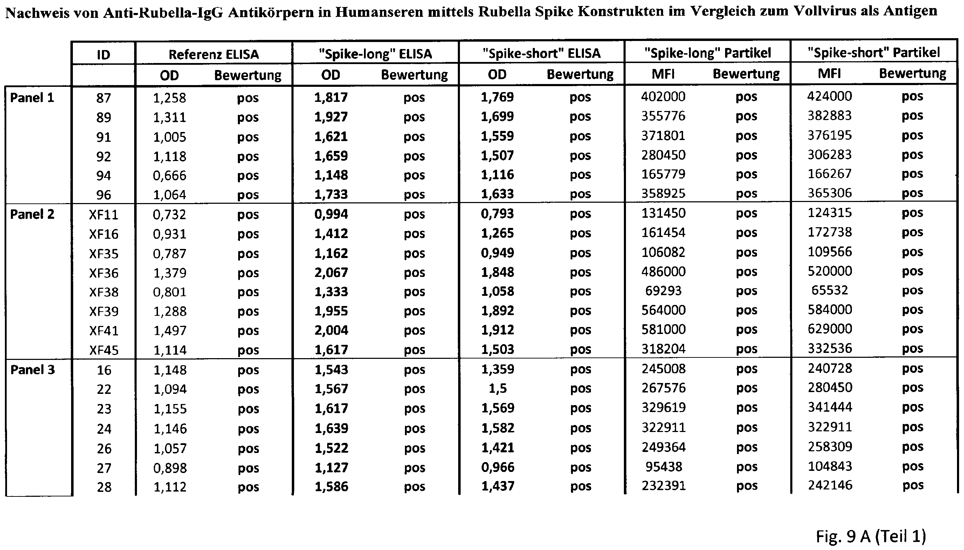

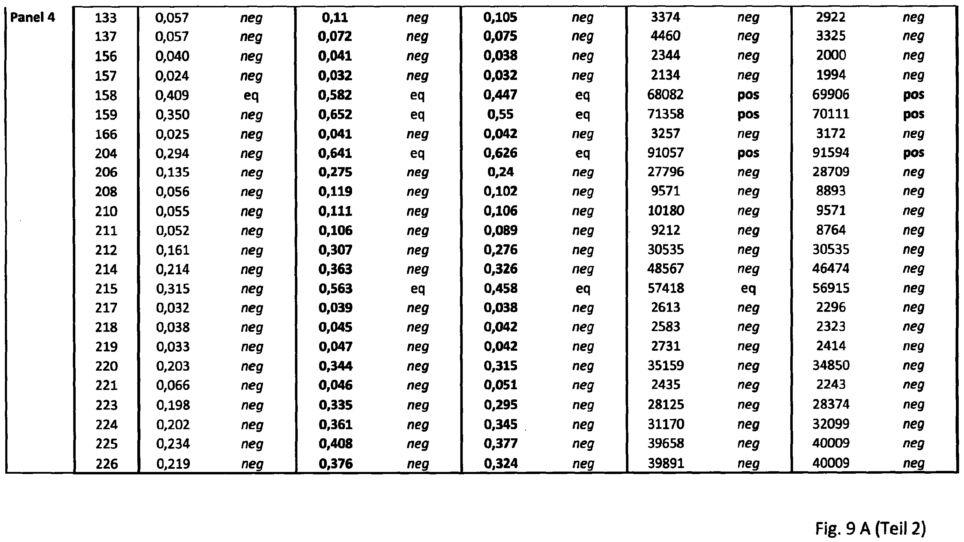

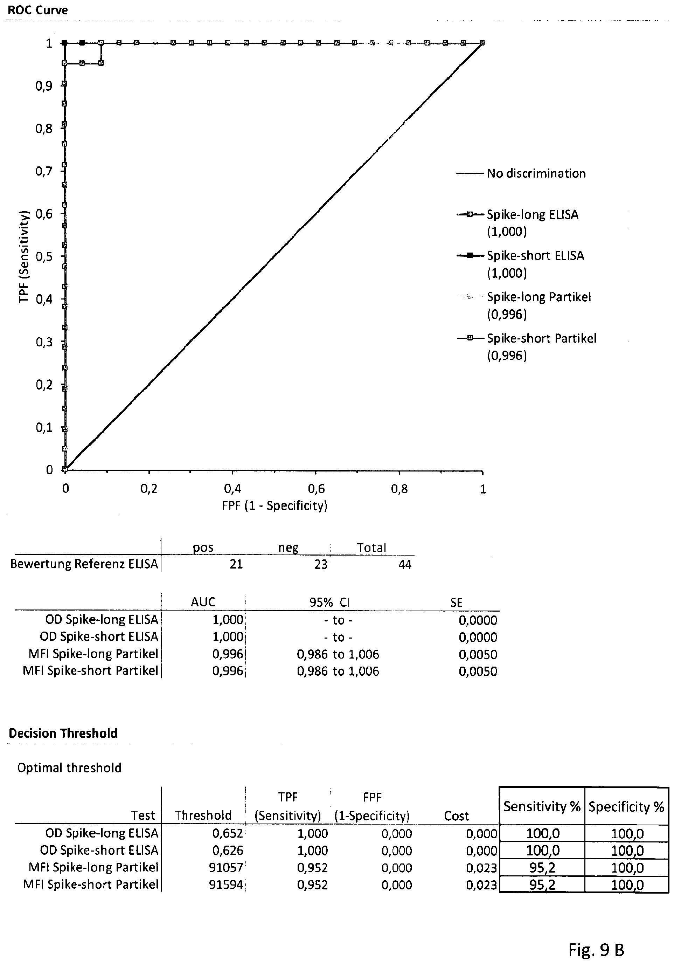

Kurzbeschreibung der Abbildungen:

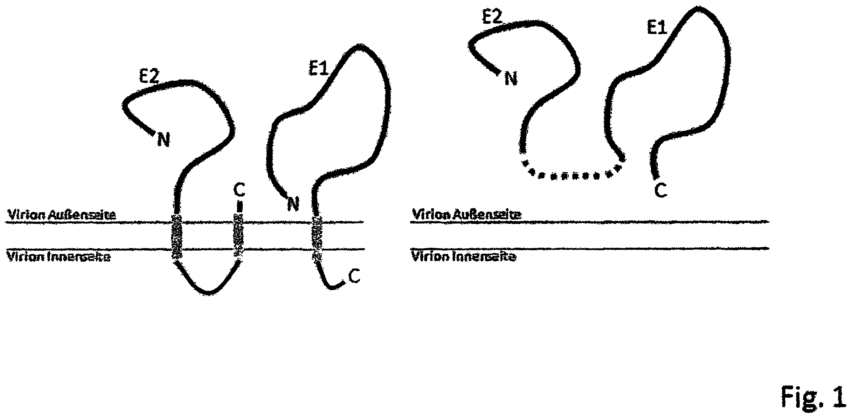

- Fig. 1:: Modelle der Glykoprotein E2/E1 Spike-Konfiguration.
(A) Konfiguration der Glykoproteine E2 und E1 auf der Oberfläche eines nativen Rubella-Virus.
(B) Konfiguration der löslichen Rubella-Spike-Konstrukte. In diesen Konstrukten werden die Transmembranregionen und die intravirionalen Domänen (hellgraue Linien) entfernt und die Ektodomänen von E1 und E2 (schwarze Linien) durch einen flexiblen Glycin-Serin-Linker verbunden.

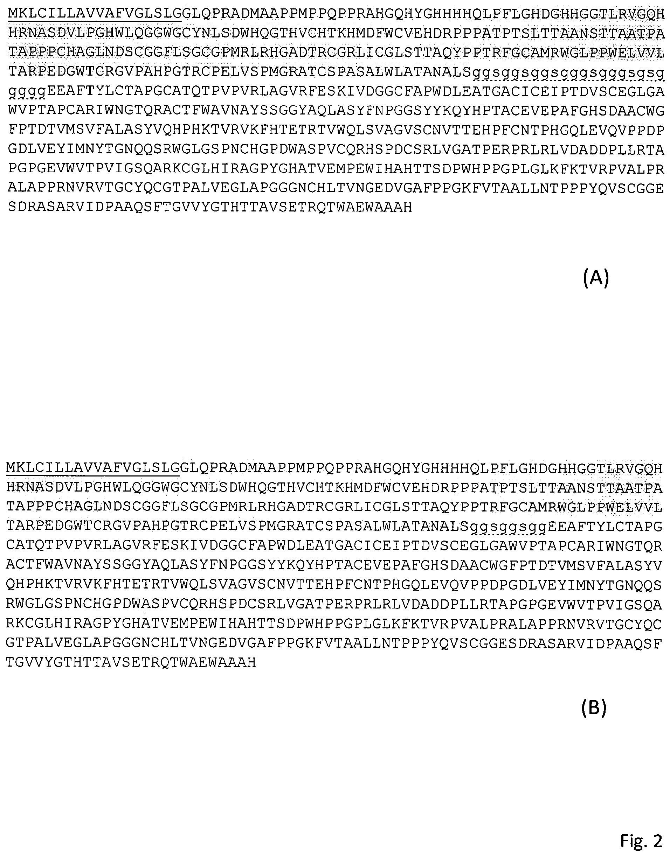

- Fig. 2:: Aminosäuresequenz des Rubella-Spike-Konstrukts;
(A) Rubella-Spike Konstrukt "long" (d.h. mit Lang-Linker)
(B) Rubella-Spike Konstrukt "short" (d.h. mit Kurz-Linker)
   Glatt Unterstrichen: BiP Signalsequenz
   Hellgrau hinterlegt: Aminosäuresequenz des E2-Fragments
   Punktiert Unterstrichen: Glycin-Serin-Linker short (8 Aminosäuren)
   Weiß hinterlegt: Aminosäuresequenz des E1-Fragments
- Fig. 3:: Charakterisierung der Rubella-Spike-Konstrukte auf SDS-PAGE:
Aliquots der aufgereinigten Rubella-Spike Konstrukte ("short" und "long") nach Auftrennung mittels SDS-PAGE (4-20% Tris-Glycin-Gele, Artikelnummer TG 42010, Anamed Elektrophorese GmbH) unter reduzierende Bedingungen und anschließender Anfärbung mit Coomassie-Brillant-Blau.
Bahn 1: Molekülgrößenmarker (kDa),
Bahn 2: Rubella-Spike-Konstrukt "long",
Bahn 3: Rubella-Spike-Konstrukt "short".

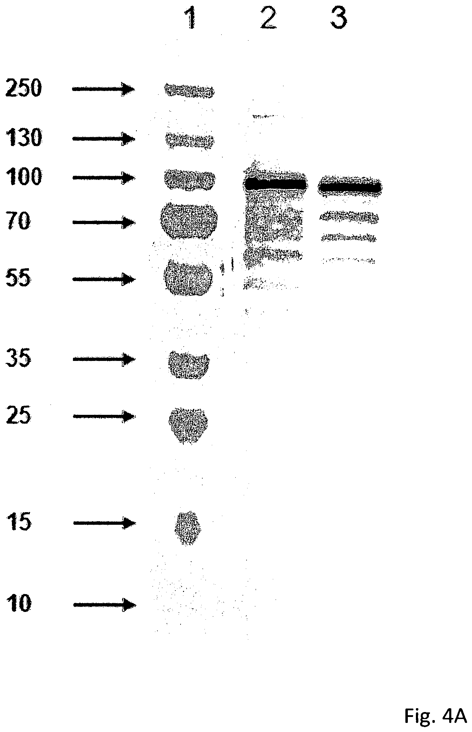

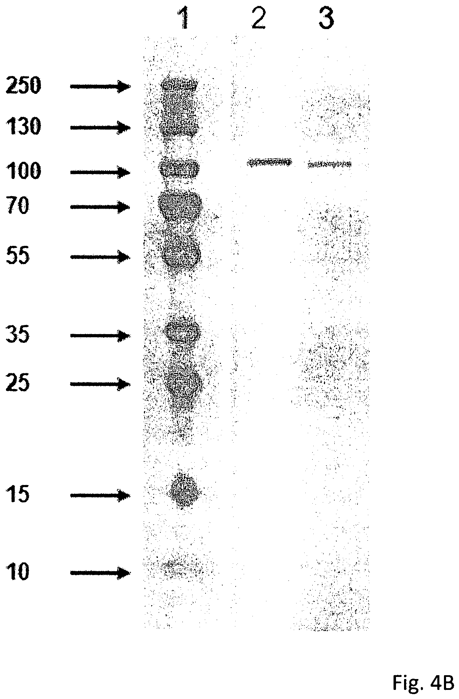

- Fig. 4:: Charakterisierung der Rubella-Spike-Konstrukte im Westernblot: Reaktion der mittels SDS-PAGE aufgereinigten und danach auf PVDF-Membran übertragenen Rubella-Spike-Proteinkonstrukte mit monoklonalen Antikörpern, die entweder gegen das Rubella-Glykoprotein E1 (A) oder gegen das Rubella-Glykoprotein E2 (B) gerichtet sind.
(A): Antikörper = Anti-Rubella-Virus Structural glycoprotein, monoclonal, mouse (MABR23-Ru6, ibt - immunological and biochemical testsystems GmbH).
   Bahn 1: Molekülgrößenmarker (kDa),
   Bahn 2: Rubella-Spike Konstrukt "long",
   Bahn 3: Rubella-Spike Konstrukt "short".
(B): Antikörper = Anti-Rubella-Virus E2 Monoclonal Antibody (D92G) (MA5-18255, Thermo Fisher Scientific).
   Bahn 1: Molekülgrößenmarker (kDa),
   Bahn 2: Rubella-Spike Konstrukt "long",
   Bahn 3: Rubella-Spike Konstrukt "short".
- Fig. 5:: Immunologische Reaktivität verschiedener Rubella-Spike-Konstrukte (als Antigene) im Vergleich mit Vollvirus - im Partikeltest mit unterschiedlichen Rubella-IgM positiven/negativen Humanseren.
x-Achse: Humanseren
y-Achse: MFI = Mean Fluorescene Intensity = Mittlere Fluoreszenzintensität
- Fig. 6:: Immunologische Reaktivität verschiedener Rubella-Spike-Konstrukte (als Antigene) im Vergleich mit Vollvirus - Titrationsverhalten im Partikeltest mit einem ausgewählten, für Rubella-IgM positiven, hoch-titrigen Humanserum.
- Fig. 7:: Immunologischen Reaktivität verschiedener Rubella-Spike-Konstrukte (als Antigene) im Vergleich mit Vollvirus - p/n (positiv/negativ) Verhältnis im partikelbasierten Nachweis von Rubella-IgM Antikörpern in verschiedenen Humanseren.
- Fig. 8:: Immunologische Reaktivität verschiedener Rubella-Spike-Konstrukte als Antigene im immundiagnostischen Test - Nachweis von Anti-Rubella-IgM Antikörpern in ausgewählten, vorcharakterisierten anti-Rubella-IgM Humanseren im Vergleich zu einem auf Vollvirus basierenden Referenztest (ELISA).
(A) (Teil 1 und Teil 2) : Wertetabelle
   ID: Probenkennung
   OD: Optischen Dichte bei 405 nm
   MFI: Mittlere Fluoreszenzintensität
   pos: positive Bewertung für anti-Rubella IgM Antikörper
   neg: negative Bewertung für anti-Rubella IgM Antikörper Fig. 8 (A) - Teil 2 ist die Fortsetzung der Tabelle Fig. 8 (A) - Teil 1 in vertikaler Richtung.
(B) : Diagnostische Performance (Diagnoseleistungsfähigkeit) - ROC (receiver operating characteristic) Kurve
- Fig. 9:: Immunologische Reaktivität verschiedener Rubella-Spike-Konstrukte als Antigene im immundiagnostischen Test - Nachweis von Anti-Rubella-IgG Antikörpern in ausgewählten, vorcharakterisierten anti-Rubella IgG Humanseren im Vergleich zu einem auf Vollvirus basierenden Referenztest (ELISA).
(A) (Teil 1 und Teil 2) : Wertetabelle
   ID: Probenkennung
   OD: Optischen Dichte bei 405 nm
   MFI: Mittlere Fluoreszenzintensität
   pos: positive Bewertung für anti-Rubella IgG Antikörper
   neg: negative Bewertung für anti-Rubella IgG Antikörper Fig. 9 (A) - Teil 2 ist die Fortsetzung der Tabelle Fig. 9 (A) - Teil 1 in vertikaler Richtung.
(B) : Diagnostische Performance (Diagnoseleistungsfähigkeit) - ROC (receiver operating characteristic) Kurve

### Beispiel 1: Generierung der erfindungsgemäßen löslichen rekombinanten Rubella-Spike-Konstrukte

Die Herstellung der erfindungsgemäßen Rubella-Spike-Konstrukte erfolgt prinzipiell derart, dass gemäß der schematischen Darstellung in Fig. 1 die Transmembranregion und die intravirionale Domäne des E2-Glykoproteins und die C-terminale Transmembranregion des E1-Glykoproteins jeweils entfernt werden und die Ektodomänen von E1 und E2 durch einen flexiblen Glycin-Serin-Linker miteinander verbunden (gekoppelt) werden.

In einer konkreten beispielhaften Ausführungsform, bei der die erfindungsgemäßen Rubella-Spike-Konstrukte von den Glykoproteinen E1 und E2 aus dem Rubella Stamm M33 abgeleitet sind, ist der C-Terminus der Ektodomäne von E2 (Aminosäuren 1-234 von E2 gemäß SEQ ID NO:8) mit dem N-Terminus der Ektodomäne von E1 (Aminosäuren 1-446 von E1 gemäß SEQ ID NO:6) über einen Kurz-Linker (Rubella Spike "short") oder ein Lang-Linker (Rubella Spike "long") verbunden. (Referenzsequenz siehe: UniProtKB/SwissProt - P08563 (POLS_RUBVM), Version 2 der Sequenz, Sequenz Update May 30, 2006). Die Aminosäuresequenz dieser Rubella-Spike-Konstrukte ist in Fig. 2A und SEQ ID NO:2 (Rubella-Spike-Konstrukt "long" d.h. mit Lang-Linker) und in Fig. 2B und SEQ ID NO:4 (Rubella-Spike-Konstrukt "short" d.h. mit Kurz-Linker) angegeben.

Bei Ableitung der erfindungsgemäßen Rubella-Spike-Konstrukte von den Glykoproteinen E1 und E2 aus dem Rubella-Stamm Rubella TO-336 (RUBV) UniProtKB/SwissProt - P08564 (POLS_RUBVV) Version 3 der Sequenz, Sequenz Update May 30, 2006, der eine 99%ige Aminosäuresequenzhomologie zum Stamm M33 aufweist, ist das Konstrukt prinzipiell genauso aufgebaut.

Für eine Expression des Rubella-Spike-Konstrukts in Drosophila melanogaster Schneider 2 (S2) Zellen sollte das Proteinkonstrukt N-terminal vorzugsweise mit einer BiP Drosophila-Signalsequenz oder eine funktionsanalog Signalsequenz versehen sein. Die BiP Drosophila-Signalsequenz fördert die Sekretion der exprimierten Rubella-Spikes aus den Drosophila S2-Zellen besonders gut.

Die Produktion der Rubella-Spikes ist nicht auf stabile oder transiente transfizierte Drosophila S2-Zellen beschränkt. Andere eukaryotische Expressionssysteme wie z.B. das Baculovirus-Expressionssystem kommen ebenfalls in Betracht. Bei Verwendung anderer Expressionssysteme können andere Signalsequenzen erforderlich sein, z.B. die gp64- oder HMS-Signalsequenz für das Baculovirus-Expressionssystem.

Um eine effektive Aufreinigung der exprimierten Rubella-Spike-Konstrukte zu ermöglichen, wird im vorliegenden Ausführungsbeispiel das erfindungsgemäße E1-E2-Fusionsprotein C-terminal mit einem Streptavidin-Affinitäts-Tag versehen (gekoppelt). Andere Affinitäts-Tags, z.B. ein His-Tag, kommen ebenfalls in Betracht. Auch die Position des Affinitäts-Tag ist nicht auf den C-Terminus des E1-E2-Fusionsproteins beschränkt, sondern kann sich auch am N-Terminus oder im Linker befinden.

### (A) Herstellung der Nukleotidsequenz, die für die Rubella-Spike-Konstrukte kodiert

Die Herstellung der Nukleotidsequenz, die für das erfindungsgemäße E1-E2-Fusionsprotein des Rubella-Spike-Konstrukts gemäß Fig. 1 (B) kodiert, erfolgt vorzugsweise anhand bekannter und in Sequenzdatenbanken hinterlegter Rubella-Virus-RNA-Sequenzen für die Hüllproteine E1 und E2.

Beispielsweise wird für das vorstehend genannte, vom Rubella-Stamm M33 abgeleitete Rubella-Spike-Konstrukt eine Nukleotidsequenz verwendet, die anhand der Rubella-Virus 24S mRNA gemäß GenBank, Accession X05259, VersionX05259.1., konstruiert wird.

Im Detail wird hierfür wie folgt vorgegangen:
Die Herstellung der Rubella-Spike Konstrukte "short" (mit Kurz-Linker) und "long" (mit Lang-Linker) Konstrukte erfolgt prinzipiell auf die gleiche Weise. Deshalb wird in der nachstehenden Beschreibung des Herstellungsverfahrens nur von Rubella-Spike-Konstrukt gesprochen.

Die Nukleotidsequenz, die für das Rubella-Spike-Konstrukt kodiert (z.B. SEQ ID NO: 1 oder SEQ ID NO:3), wird synthetisch hergestellt, vorzugsweise noch um eine Affinitäts-Tag-Sequenz ergänzt, und in einen Standard pMX-Klonierungsvektor (Invitrogen/Geneart, Regensburg) kloniert. Sie ist für die Expression in *Drosophila melanogaster* kodonoptimiert und enthält eine Kozak-Sequenz (gccaccATG), um einen effizienten Start der Translation im Rahmen der Proteinbiosynthese in Drosophila S2-Zellen zu gewährleisten. Zusätzlich enthält das DNA-Konstrukt eine EcoRI und eine NotI Restriktionsschnittstelle zum Zweck der Klonierung des Rubella-Spike-DNA-Konstrukts in den Expressionsvektor pExpres2.1 (vgl. EP2307543B1; im Handel erhältlich bei Expres2ion Biotechnologies, Horsholm, Dänemark).

### (B) Herstellung des Transfektionsvektors für die rekombinante Proteinproduktion

Gemäß (A) hergestellte Nukleotidsequenzen, die für Rubella-Spike-Konstrukte kodieren, - hier beispielsweise für aus Rubella-Stamm M33 abgeleitete Rubella-Spike-Konstrukte (z.B. SEQ ID NO:1 oder SEQ ID NO:3) -, werden in einen Transfektionsvektor kloniert, der für die Zellen des vorgesehenen Zellkultursystems geeignet sind.

Als Zellkultursystem kommen insbesondere Drosophila melanogaster Schneider 2 (S2) Zellen in Betracht. Ein für diese Zellen geeigneter Transfektionsvektor ist der Drosophila S2 Expressionsvektor pExpres2.1 (im Handel erhältlich bei Expres2ion Biotechnologies, Horsholm, Dänemark; siehe auch EP2307543B1). Als Selektionsmarker enthält der pExpres2.1-Expressionsvektor das Zeocin Resistenzgen.

Für die Expression in den Drosophila (S2)-Zellen gemäß vorliegendem Ausführungsbeispiel wurde die gemäß Beispiel (A) hergestellte (für die Rubella-Spike-Konstrukte kodierende) Nukleotidsequenz in den Expressionsvektor pExpres2.1 insertiert.

Die hierzu erforderlichen Klonierungsschritte wurden gemäß pExpres2.1-Herstellerangaben durchgeführt und sind dem Fachmann prinzipiell bekannt und geläufig.

Mit dem derart generierten Transfektionsvektor wurden Drosophila S2-Zellen transfiziert.

### (C) Expression der Rubella-Spike-Konstrukte in Drosophila S2 Zellkulturen

Drosophila S2-Zellen sind Zelle aus der embryonalen Schneider-2-Zelllinie Drosophila melanogaster, die u.a. bei der DSMZ - Deutsche Sammlung von Mikroorganismen und Zellkulturen GmbH, Inhoffenstraße 7 B, 38124 Braunschweig, Deutschland unter der Depotnummer DSMZ ACC 130, und bei der American Type Culture Collection (ATCC), Postfach 1549, Manassas, VA 20108, USA, unter der Depotnummer CRL-1963, hinterlegt sind und von dort bezogen werden können.

Die in dem hier beschriebenen Ausführungsbeispiel eingesetzten Drosophila S2-Zellen stammen aus Zellkulturen der Firma Expres2ion Biotechnologies, Horsholm, Dänemark, sie sind im Handel erhältlich als sogenannte "ExpreS² cells", und sie werden im folgenden kurz "S2-Zellen" genannt.

### Verwendete Materialien:

- Fötales Kälberserum "FKS"
- Serum-freies Medium für Insektenzellen, z.B. EX-CELL^{®} 420 (Artikelnummer 14420, Sigma)
- Zeocin (Artikelnummer R25001, Thermo Fisher Scientific)
- Penicillin-Streptomycin, 10,000 U/ml Penicillin, 10 mg/ml Streptomycin (Artikelnummer P06-07050, PAN Biotech)
- (Drosophila melanogaster) S2-Zellen (ExpreS² cells, Expres2ion Biotechnologies)
- ExpreS² Insect-TRx5 Transfektionsreagenz (Artikelnummer S2-55A-001, Expres2ion Biotechnologies)
- Puffer W: 100 mM Tris/HCl pH8,0, 150 mM NaCl, 1 mM EDTA
- Puffer E: 100 mM Tris/HCl pH8,0, 150 mM NaCl, 1 mM EDTA, 2,5 mM Desthiobiotin
- BioLock-Biotin Blocking Solution (Artikelnummer 2-0205-050, IBA Lifesciences)
- Zellkulturflasche mit Filterkappe, 25 cm² (T-25) (Artikelnummer 156367, Thermo Fisher Scientific)
- Zellkulturflasche mit Filterkappe, 80 cm² (T-80) (Artikelnummer 178905, Thermo Fisher Scientific)
- Schüttelflasche mit Filterkappe, 250 mL (Artikelnummer 431144, Corning) (Arbeitsvolumen: 20-60 mL)
- Rollerflasche, PET 850 cm² (Artikelnummer 680180, Greiner bio-one GmbH) mit Lüftungsdeckel (Artikelnummer 383382, Greiner bio-one GmbH) (Arbeitsvolumen: 200-400 mL)
- Kryoröhrchen mit Innengewinde (Artikelnummer 114841, Brand GmbH)
- Zellzählkammer, Neubauer Improved (Brand GmbH)
- Schüttelinkubator (CERTOMAT BS-1, Sartorius Stedim Biotech)
- TFF-Membran, Vivaflow 200, 10.000 MWCO Hydrosart Membran (Artikelnummer VF20H0, Sartorius Stedim Biotech)
- Filter (0,45 µm), Minisart (Artikelnummer 16555-K, Sartorius Stedim Biotech)
- Äkta-pure Chromatographieanlage (GE Healthcare)
- Strep-Tactin^{®} Superflow^{®} high capacity cartridge, 5 mL (Artikelnummer 2-1238-001, IBA Lifesciences)

### (i) Transfektion und Herstellung einer stabil transformierten Drosophila S2 Zelllinie

Zur Herstellung der stabil transformierten S2-Zelllinie werden die gemäß (B) hergestellten pExpres2-1 Expressionsvektoren mit den für die Rubella-Spike-Konstrukte ("short" oder "long") kodierenden Inserts in die S2-Zellen transfektiert. Hierfür werden die S2-Zellen der Schüttelflasche gezählt und auf 2 × 10⁶ Zellen/mL in EX-CELL420 Medium eingestellt. Pro Transfektion werden 5 mL dieser Zellsuspension in eine T-25 Zellkulturflasche überführt. Anschließend werden 50 µL Transfektionsreagenz zugegeben und das Transfektionsreagenz durch Kippen der Flasche gleichmäßig im Medium verteilt. Dann wird die Plasmid-DNA (vorzugsweise 5-15 µg) zugegeben und durch Kippen der Flasche gleichmäßig verteilt. Die T-25 Flasche werden bei 23-27°C inkubiert. Nach etwa 3-4 Stunden wird 1 mL FKS zugegeben.

Nach 2 Tagen wird die Selektionsphase eingeleitet, indem Zeocin bis zu einer Endkonzentration von etwa 1500-2000 µg/mL zugegeben wird. Die Zellen werden alle 3-4 Tage gezählt. Sobald ihre Konzentration höher als 1 × 10⁶ Zellen/mL ist, werden die Zellen in frischem Selektionsmedium (EX-CELL420 + 10% FKS und Zeocin (Endkonzentration etwa 1500-2000 µg/mL)) auf 1 × 10⁶ Zellen/mL in einem Endvolumen von 6 mL ausgedünnt. Dieser Vorgang wird mit mehrtägigem Abstand mehrmals wiederholt.

Nach 2-4 Wochen Selektion in Zeocin haltigem Medium kann die Zelllinie als stabil angesehen werden. Im Anschluss an die Selektionsphase und sobald die Zellen eine Konzentration von >6 × 10⁶ Zellen/mL erreicht haben, werden 6 mL dieser Zellsuspension von der T-25 in eine T75 Flasche mit 4 mL frischem Medium (EX-CELL420 + 10 % FKS) überführt. Sobald sich diese Zellen erholt haben, wird nochmals 5 mL frisches Medium (EX-CELL420 + 10% FKS) zugegeben. Nach 3-4 Tage werden die Zellen gezählt. Sobald die Zellen eine Konzentration von >6 × 10⁶ Zellen/mL erreicht haben, werden 15 mL dieser Zellen in eine 250 mL Schüttelflasche überführt und 15 mL Medium (EX-CELL420) zugefügt. Die Expandierung der Zellen erfolgt wie vorstehend unter (ii) beschrieben.

### (ii) Expansion der Drosophila S2 Zellen in Schüttelflaschen

Die gemäß (i) hergestellte S2-Zellkultur wird im Expandierungsprozess gehalten und überwacht. Sobald die Viabilität der Zellen >90% und die Zellkonzentration >8 × 10⁶ Zellen/mL beträgt, werden die Zellen in EX-CELL420 Medium auf 8×10⁶ Zellen/mL ausgedünnt. Sofern das Gesamtvolumen der Kultur das zulässige Arbeitsvolumen der Schüttelflasche dabei nicht übersteigt, wird zu diesem Zweck das frische Medium der Ursprungsflasche einfach zugegeben. Falls das Gesamtvolumen das zulässige Arbeitsvolumen der Ursprungsflasche überschreiten würde, werden die Zellen auf 8 × 10⁶ Zellen/mL ausgedünnt und auf mehrere Schüttelkolben verteilt. Typischerweise werden die Zellen alle 3-4 Tage ausgedünnt. Nach 3-4 Tagen ist typischerweise eine Zellkonzentration von 25-50 × 10⁶ Zellen/mL erreicht.

### (iii) Produktion der Rubella-Spike-Konstrukte

Für die Produktion und Gewinnung der Rubella-Spike-Konstrukte werden die gemäß (i) oder (ii) erhaltenen polyklonalen Zelllinien in serumfreiem Medium kultiviert. Nach etwa vier Tagen Kultivierung werden die Zellen durch Zentrifugation entfernt und der Überstand geerntet. Aus dem Überstand werden die Rubella-Spike-Konstrukte anhand ihrer C-terminalen Streptavidin-Affinitäts-Tag-Markierung mittels Affinitätschromatographie über Strep-Tactin-Säulen isoliert.

Im Einzelnen wird hierfür beispielsweise wie folgt vorgegangen:
Die Zelldichte der in (i) oder (ii) erhaltenen Zellsuspension wird auf 8 × 10⁶ Zellen/mL eingestellt, und die Zellen werden unter Zugabe von Penicillin-Streptomycin (1:000) in Rollerflasche (mit Lüftungsdeckel) überführt. Die Rollerflaschen werden stehend in einem Schüttelinkubator bei 27°C und 120 rpm für 4 Tage inkubiert. Danach werden die Zellen durch Zentrifugation (4.400 × g, 20 min) abgetrennt und entfernt. Der Zellüberstand wird zuerst mittels Tangentialfluss-Filtration (Vivaflow 200, 10.000 MWCO Hydrosart Membran) auf 1/10 des Anfangsvolumen aufkonzentriert und anschließend mit der gleichen Membran gegen das zehnfache Volumen Puffer W kontinuierlich diafiltriert. Zur Inhibition von Proteasen im aufkonzentrierten Zellüberstand wird PMSF (Endkonzentration 1 mM) zugefügt. Um Biotin aus dem aufkonzentrierten Zellüberstand zu entfernen, wird eine BioLock Biotin blocking solution (3 mL/L) zugefügt, 15 min unter Rühren bei Raumtemperatur inkubiert und anschließend zentrifugiert (10.000 × g, 30 min, 4°C). Der Überstand wird mit einem 0,45 µm Filter filtriert. Zur Aufreinigung der Strep-getaggten Rubella-Spike-Konstrukte wird der Überstand auf eine Strep-Tactin Superflow Säule einer Äkta-pure Chromatographieanlage aufgetragen. Nach dem Auftragen wird mit 4 Säulenvolumen Puffer W gewaschen und anschließend mit 5 Säulenvolumen Puffer E eluiert. Das erhaltene Eluat wird in mehreren separaten Fraktionen gesammelt. Die Fraktionen, die das Rubella-Spike-Konstrukt enthalten, werden vereinigt und eine Proteinkonzentrationsbestimmung (z.B. nach Bradford) durchgeführt.

### Beispiel 2: Charakterisierung der antigenen Eigenschaften der Rubella-Spike-Konstrukte mittels SDS-PAGE und Westernblot.

Aliquote der gemäß Beispiel 1 aufgereinigten Rubella-Spike-Konstrukte "short" und "long" werden auf SDS-PAGE (4-20% Tris-Glycin-Gele, Artikelnummer TG 42010, Anamed Elektrophorese GmbH) aufgetragen, unter reduzierenden Bedingungen aufgetrennt und anschließend mit Coomassie-Brillant-Blau angefärbt. Die Ergebnisse sind in Fig. 3 abgebildet. Aus dem Vergleich mit den Molekülgrößenmarkerbanden (kDa) in Bahn 1 wird ersichtlich, dass Rubella-Spike Konstrukt "Long" (Bahn 2) und Rubella-Spike-Konstrukt "Short" (Bahn 3) Molekülgrößen von etwa 80 kDa aufweisen. Diese Werte stimmen mit den Molekülgrößen überein, die rechnerisch auf Basis deren Aminosäuresequenz zu erwarten sind, nämlich 77 kDa für Rubella-Spike-Konstrukt "Long" und 76 kDa für Rubella-Spike-Konstrukt "Short".

Für den Westernblot werden die Proteine aus der SDS-PAGE auf eine PVDF-Membran übertragen. Nach der Blockierung freier Proteinbindungsstellen werden die Proteine auf der Membran mit einem monoklonalen Antikörper für das Rubella-Glykoprotein E1 und einem monoklonalen Antikörper für das Rubella-Glykoprotein E2 inkubiert. Bei den eingesetzten Antikörpern handelt es sich beispielsweise um (a) Anti-Rubella-Virus Structural glycoprotein, monoclonal, mouse (MABR23-Ru6, ibt - immunological and biochemical testsystems GmbH), der mit Glykoprotein E1 reagiert, und (b) Rubella-Virus E2 Monoclonal Antibody (D92G) (MA5-18255, Thermo Fisher Scientific), der mit Glykoprotein E2 reagiert. Zur Visualisierung wird anschließend mit einem anti-Maus IgG Antikörper aus Ziege (Alkaline Phosphatase Konjugat) inkubiert. Die Ergebnisse der Westernblots sind in Fig. 4 (A) und (B) abgebildet. Aus dem Vergleich mit den Molekülgrößenmarkerbanden (kDa) in Bahn 1 wird ersichtlich, dass sowohl das Rubella-Spike-Konstrukt "long" (Bahn 2) als auch das Rubella-Spike-Konstrukt "short" (Bahn 3) von den Anti-Rubella E1- und Anti-Rubella E2-Antikörpern erkannt werden. Diese Ergebnisse zeigen eindeutig, dass sowohl im Konstrukt "long" als auch im Konstrukt "short" Epitope von Rubella-Glykoprotein E1 und Rubella-Glykoprotein E2 vorhanden sind.

### Beispiel 3: Untersuchung der immunologischen Reaktivität der Rubella-Spike-Konstrukte im immundiagnostischen Test: Nachweis von Anti-Rubella-IgM Antikörpern in Humanseren

Hierfür wurden zwei verschiedene Testsysteme eingesetzt - ein konventionelles ELISA System und ein Partikel-basiertes System, welches auf Durchflusszytometrie und einem Fluoreszenz-Read-Out des Konjugatsignals beruht. Beide Systeme (ELISA und Partikelbasiert) sind repräsentative Systeme (Antigenträger - Partikel oder Platte) für aktuell gängige Nachweisverfahren in der Rubella IgM und IgG Serologie.

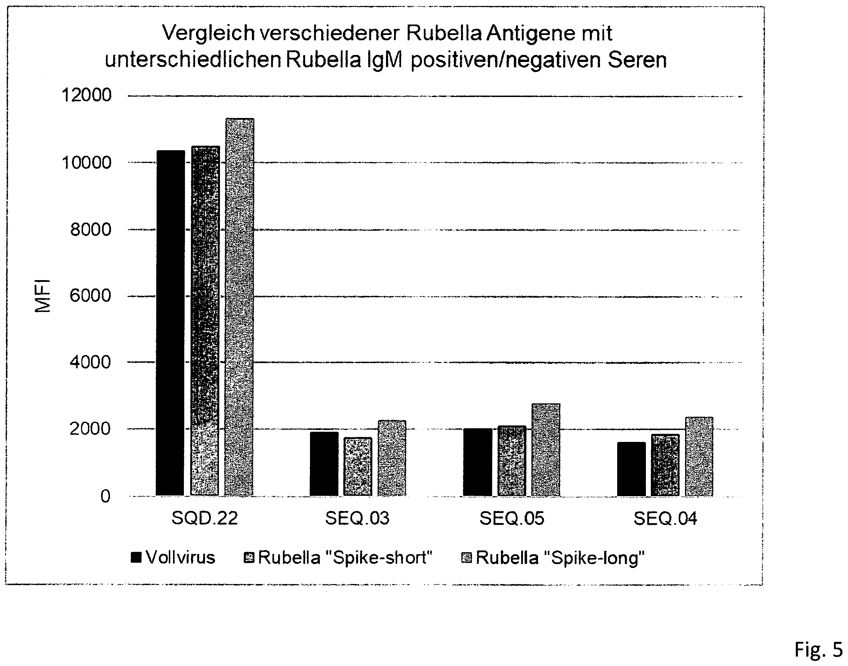

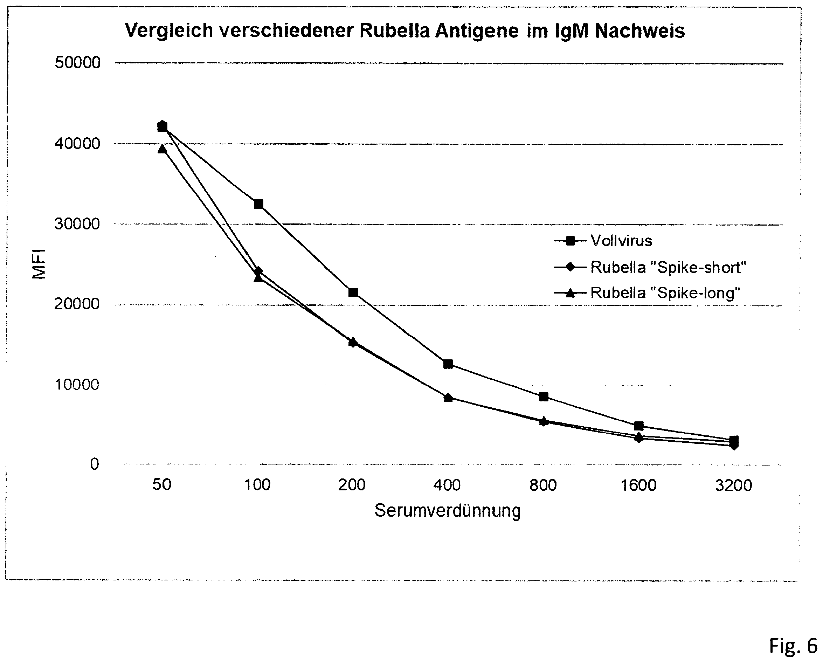

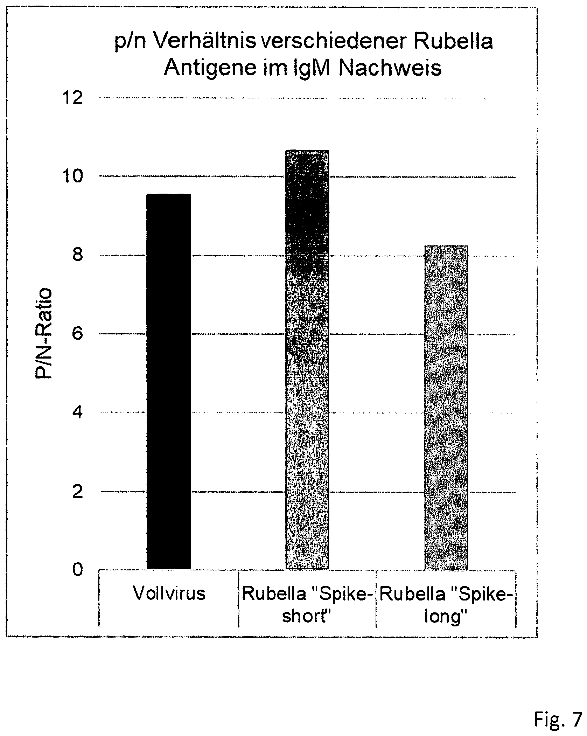

Für die Untersuchung der immunologischen Reaktivität der Rubella-Spike-Konstrukte hinsichtlich Patienten-IgM-Antikörper wurden verschiedene Testläufe jeweils im Vergleich mit dem Vollvirus (als dem im Stand der Technik gebräuchlichen Antigen) durchgeführt:
(a) Ein Partikeltest mit vier verschiedenen Humanseren (d.h. Seren von vier verschiedenen Patienten) lieferte die in Fig. 5 graphisch dargestellten Ergebnisse. Die "Mean Fluorescene Intensity (MFI)" auf der y-Achse ist ein Maß für die Menge an detektierten Patienten IgM-Antikörpern in der jeweiligen Probe. Die Ergebnisse demonstrieren, dass die erfindungsgemäßen Rubella-Spike-Kontrukte "short" und "long" die Detektionsleistung des Vollvirus mindestens erreichen, in den meisten Fällen sogar überragen.
(b) Ein Partikeltest mit verschiedenen Verdünnungen eines ausgewählten, für Rubella IgM positiven und hoch-titrigen Humanserums lieferte die in Fig. 6 graphisch dargestellten Ergebnisse. Die Mean Fluorescene Intensity (MFI) auf der y-Achse ist ein Maß für die Menge an detektierten Patienten IgM-Antikörpern in der jeweiligen Verdünnung der Probe (x-Achse). Die Ergebnisse demonstrieren, dass die erfindungsgemäßen Rubella-Spike-Kontrukte "short" und "long" mit einem Humanserum im Vergleich zum Vollvirus ein sehr ähnliches, von der Form her sogar ein verbessertes "Titrationsverhalten" zeigen. Dies ist eine essenzielle Eigenschaft eines Antigens für einen serologischen Test.
(c) Das positiv-zu-negativ-Verhältnisses (P/N) wurde mit Hilfe dem Fachmann bekannter Methoden unter Anwendung eines Partikel-basierten in-house Verfahrens und unter Einsatz verschiedener für Rubella IgM positiver und negativer Humanseren für die Antigene Rubella-Spike-Konstrukt "long" und "short" jeweils im Vergleich zum Vollvirus bestimmt. Für jedes Antigen bzw. Testsetup wurde das entsprechende positiv-zu-negativ-Verhältnisses (P/N) ermittelt. Die Ergebnisse sind in Fig. 7 graphisch dargestellt. Sie demonstrieren, dass die erfindungsgemäßen Rubella-Spike-Kontrukte "short" und "long" in einem serologischen Test zum Nachweis von Rubella IgM Antikörpern ein dem Vollvirus sehr ähnliches, teilweise überlegenes positiv-zu-negativ-Verhältnisses (P/N) aufweisen. Das positiv-zu-negativ-Verhältnisses (P/N) eines diagnostischen Tests ist, wie auch das Titrationsverhalten, eine essenzielle Kenngröße dieses Testverfahrens und ein Maß für die sogenannte diagnostische Leistungsfähigkeit: Je größer der Quotient P/N desto größer bzw. besser die diagnostische Leistungsfähigkeit.
(d) In einem zusätzlichen Vergleichstest wurden die beiden verschiedenen Testsysteme ELISA und Partikel-basiertes System für die Untersuchung der immunologischen Reaktivität der Rubella-Spike-Konstrukte "short" und "long" hinsichtlich Anti-Rubella-IgM Antikörper in 36 vorcharakterisierte (d.h. bezüglich Anti-Rubella IgM Antikörper vorab analysierte) Humanserumproben, 17 positive und 19 negative, eingesetzt. Als Referenz diente ein mit Vollvirus beschichteter ELISA.

Für den Vergleich und die Festlegung der cut-off Werte für die experimentellen Setups wurde in Microsoft Excel eine ROC-Analyse mittels der Software "Analyse-it" (Analyse-it Software, Ltd., UK) durchgeführt.

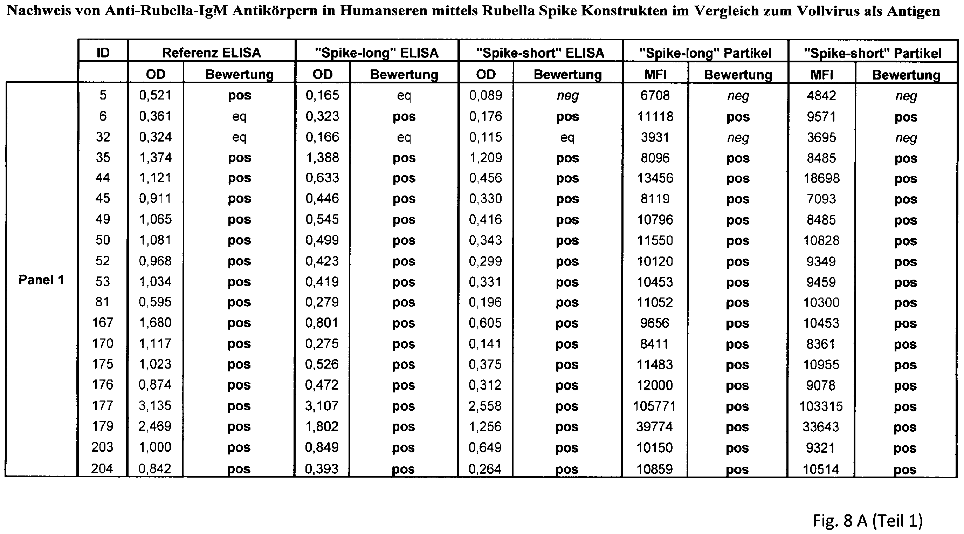

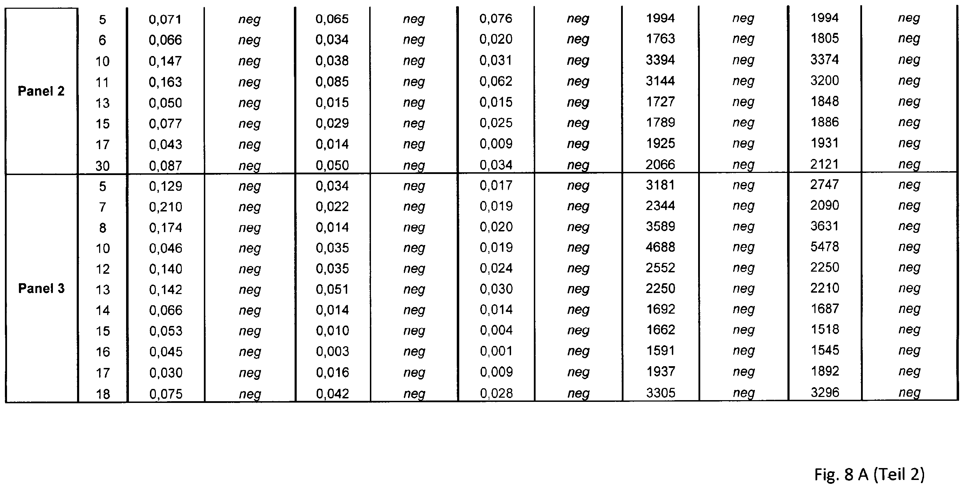

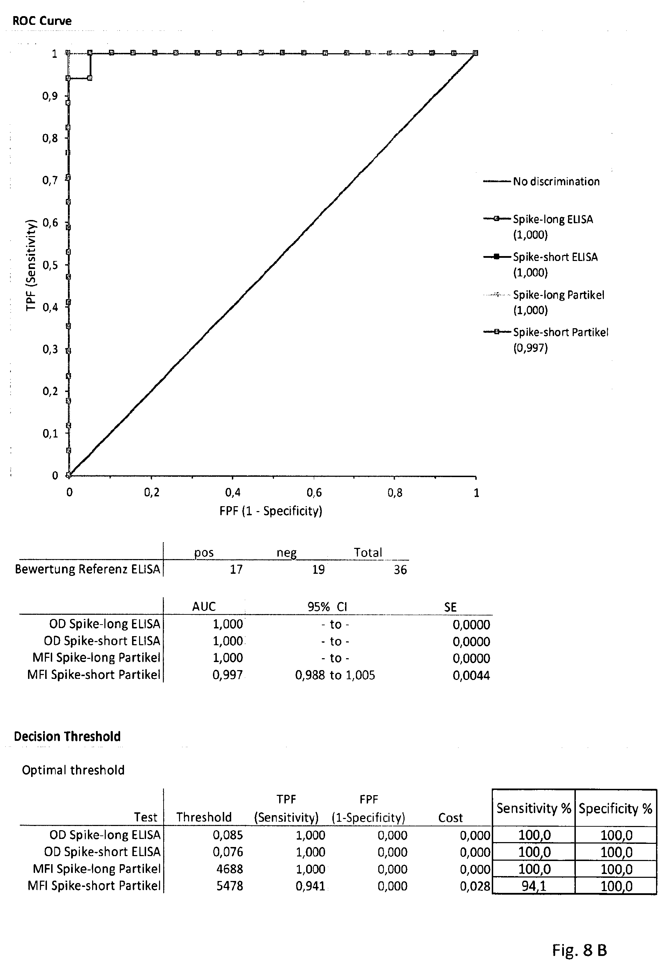

Die Testergebnisse sind in Fig. 8A und Fig. 8B tabellarisch und graphisch dargestellt.

Aus Fig. 8A und Fig. 8B ist erkennbar, dass die neuen Rubella-Spike-Konstrukt-Antigene "short" und "long" sowohl im ELISA Setup als auch im Partikel-basierten Test erfolgreich für die serologische Differenzierung von Rubella IgM positiven und Rubella IgM negativen Patientenproben eingesetzt werden können. Mit beiden Rubella-Spike-Konstrukt-Antigenen ("short" und/oder "long") ist eine eindeutige Unterscheidung von positiven und negativen Proben aus/in unterschiedlichen Spenderkollektiven (Panel) möglich. Im Vergleich zum Referenztest (ELISA) wurden mit dem Rubella-Spike-Konstrukt "long" auf beiden Test-Plattformen Sensitivitäten und Spezifitäten von 100% erreicht. Mit dem Rubella-Spike-Konstrukt "short" wurden im ELISA ebenfalls Sensitivitäten und Spezifitäten von 100% erreicht, im Partikel-basierten Test lag die Sensitivität bei 94,1% und die Spezifitäten auch wieder bei 100%.

### Beispiel 4: Untersuchung der immunologischen Reaktivität der Rubella-Spike-Konstrukte im immundiagnostischen Test: Nachweis von Anti-Rubella-IgG Antikörpern in Humanseren

Hierfür wurden wie in Beispiel 3 zwei verschiedene Testsysteme eingesetzt - ein konventionelles ELISA System und ein Partikel-basiertes System, das auf Durchflusszytometrie und einem Fluoreszenz-Read-Out des Konjugatsignals beruht.

Für die Untersuchung der immunologischen Reaktivität der Rubella-Spike-Konstrukte "short" und "long" hinsichtlich Anti-Rubella-IgG Antikörper wurden 44 vorcharakterisierte (d.h. bezüglich Anti-Rubella IgG Antikörper vorab analysierte) Humanserumproben, 21 positive und 23 negative, auf den beiden genannten Plattformen gegen einen kommerziellen Referenz ELISA ausgetestet. Der Referenz ELISA war mit Vollvirus beschichtet.

Für den Vergleich und die Festlegung der cut-off Werte für die experimentellen Setups wurde in Microsoft Excel eine ROC-Analyse mittels der Software "Analyse-it" (Analyse-it Software, Ltd., UK) durchgeführt.

Die Testergebnisse sind in Fig. 9A und Fig. 9B tabellarisch und graphisch dargestellt.

Aus Fig. 9A und Fig. 9B ist erkennbar, dass die neuen Rubella-Spike-Konstrukt-Antigene "short" und "long" sowohl im ELISA Setup, als auch im Partikel-basierten Test erfolgreich für die serologische Differenzierung von Rubella IgG positiven und Rubella IgG negativen Patientenproben eingesetzt werden können. Mit beiden Rubella-Spike-Konstrukt-Antigenen ("short" und/oder "long") ist eine eindeutige Unterscheidung von positiven und negativen Proben aus/in unterschiedlichen Spenderkollektiven (Panel) möglich. Im Vergleich zum Referenztest (ELISA) wurden mit beiden Rubella-Spike-Konstrukten "long" und "short" auf beiden Test-Plattformen Spezifitäten von 100% erreicht. Die Sensitivitäten betrugen bei beiden Konstrukten im ELISA ebenfalls jeweils 100%, im Partikel-basierten Test lag die Sensitivität bei beiden Konstrukten ebenfalls jeweils bei 95,2%.

### Zitierte Nicht-Patent-Literatur:

Battisti et al., 2012. Cryo-Electron Tomography of Rubella Virus, J Virol. 2012 Oct; 86(20): 11078-11085.
DuBois et al., 2012. Functional and evolutionary insight from the crystal structure of rubella virus protein E1. Nature. 2013 Jan 24;493(7433):552-6.
Hobman et al., 1993: The rubella virus E2 and E1 spike glycoproteins are targeted to the Golgi complex. J. Cell Biol. 121: 269-281 (1993).
Hobman et al., 2007. Fields Virology Vol. 1 (ed. D. M Knipe) 1069-1100 (Lippincott Williams & Wilkins, 2007).
Katow et al., 1988. Low pH-induced conformational change of rubella virus envelope proteins. J Gen Virol 1988;69(pt 11):2797-2807.
Perrenoud G. et al. 2004: A recombinant rubella virus E1 glycoprotein as a rubella vaccine candidate. Vaccine 2004; 23(4):480-8.
Petrova et al, 2016. The key role of rubella virus glycoproteins in the formation of immune response, and perspectives on their use in the development of new recombinant vaccines. Vaccine 2017, 34(8):1006-1011.
Prasad et al., 2017. Assembly, maturation and three-dimensional helical structure of the teratogenic rubella virus. PLoS Pathog. 2017 Jun 2;13(6).
Schneider, I., 1972. Cell lines derived from late embryonic stages of Drosophila melanogaster. Journal of Embryology and Experimental Morphology 27 (2): 353-365. http://www.ncbi.nlm.nih.gov/pubmed/4625067.
Seppänen et al. 1991: Diagnostic potential of baculovirus-expressed rubella virus envelope proteins. Clin. Microbiol, 1991, 1877-1882.
Waxham et al., 1985. Detailed immunologic analysis of the structural polypeptides of rubella virus using monoclonal antibodies. Virology 1985;143:153-165.

## Patentansprüche

1. Rubella-Virus-Antigen, **dadurch gekennzeichnet, dass** es ein Rubella Virus E1-E2-Hüllprotein-Komplex-Konstrukt (Synonym: Rubella-Spike-Konstrukt) ist, umfassend
- wenigstens eine E1-Komponente und eine E2-Komponente, die verbunden sind,
- wobei die E1-Komponente aus dem E1-Hüllprotein besteht, dessen C-terminale Transmembranregion und intravirionale Domäne entfernt sind und dessen N-Terminus die Ektodomäne des E1-Hüllproteins umfasst,
- und wobei die E2-Komponente aus dem E2-Hüllprotein besteht, dessen Transmembranregionen und intravirionale Domäne entfernt sind und dessen N-Terminus die Ektodomäne des E2-Hüllproteins umfasst,
- und wobei der C-Terminus der E2-Komponente mit dem N-Terminus der E1-Komponente unmittelbar oder mittels Linker verbunden ist.

2. Rubella-Virus-Antigen nach Anspruch 1, **dadurch gekennzeichnet, dass** das Antigen als rekombinantes E1-E2-Fusionsprotein hergestellt wird.

3. Rubella-Virus-Antigen nach Anspruch 2, **dadurch gekennzeichnet, dass** das E1-E2-Fusionsprotein mit einer Signalsequenz gekoppelt ist.

4. Rubella-Virus-Antigen nach einem der Ansprüche 1 bis 3, **dadurch gekennzeichnet, dass** das E1-E2-Fusionsprotein mit einem Affinitäts-Tag, vorzugsweise einem Streptavidin-Affinitäts-Tag gekoppelt ist, der vorzugsweise C-terminal angeordnet ist.

5. Rubella-Virus-Antigen nach einem der Ansprüche 1 bis 4, **dadurch gekennzeichnet, dass** die Ektodomäne des E1-Hüllproteins die Aminosäuren (der Positionen) 1-446 gemäß SEQ ID NO:6 oder die Aminosäuren AA583-1028 der Referenzsequenz UniProtKB/SwissProt - P08563 (POLS_RUBVM), Sequenz Update May 30, 2006 (Version 2 der Sequenz) aufweist.

6. Rubella-Virus-Antigen nach einem der Ansprüche 1 bis 5, **dadurch gekennzeichnet, dass** die Ektodomäne des E2-Hüllproteins die Aminosäuren (der Positionen) 1-234 gemäß SEQ ID NO:8 oder die Aminosäuren AA301-534 der Referenzsequenz UniProtKB/SwissProt - P08563 (POLS_RUBVM), Sequenz Update May 30, 2006 (Version 2 der Sequenz) aufweist.

7. Rubella-Virus-Antigen nach einem der Ansprüche 1 bis 6, **dadurch gekennzeichnet, dass** der Linker ein flexibler Linker ist, der Glycin und/oder Serin aufweist.

8. Rubella-Virus-Antigen nach einem der Ansprüche 2 bis 7, **dadurch gekennzeichnet, dass** das Fusionsprotein die Aminosäuresequenz gemäß SEQ ID NO: 2 umfasst, wobei die Signalsequenz am N-terminalen Ende und/oder die Linker-Sequenz im mittleren Bereich dieser Aminosäuresequenz fakultativ vorhanden ist.

9. Rubella-Virus-Antigen nach einem der Ansprüche 2 bis 7, **dadurch gekennzeichnet, dass** das Fusionsprotein die Aminosäuresequenz gemäß SEQ ID NO: 4 umfasst, wobei die Signalsequenz am N-terminalen Ende und/oder die Linker-Sequenz im mittleren Bereich dieser Aminosäuresequenz fakultativ vorhanden ist.

10. Rekombinantes DNA-Molekül, das ein Rubella-Virus-Antigen kodiert und eine für ein Rubella E1-E2-Fusionsprotein nach Anspruch 2 codierende Nukleotidsequenz umfasst.

11. Rekombinantes DNA-Molekül nach Anspruch 10, **dadurch gekennzeichnet, dass** es die Nukleotidsequenzen gemäß SEQ ID NO: 5 und SEQ ID NO: 7 umfasst.

12. Rekombinantes DNA-Molekül nach Anspruch 10 oder 11, **dadurch gekennzeichnet, dass** es die Nukleotidsequenz gemäß SEQ ID NO: 1 umfasst, wobei die Signalsequenz am N-terminalen Ende und/oder die Linker-Sequenz im mittleren Bereich dieser Nukleotidsequenz fakultativ vorhanden ist.

13. Rekombinantes DNA-Molekül nach Anspruch 10 oder 11, **dadurch gekennzeichnet, dass** es die Nukleotidsequenz gemäß SEQ ID NO: 3 umfasst, wobei die Signalsequenz am N-terminalen Ende und/oder die Linker-Sequenz im mittleren Bereich dieser Nukleotidsequenz fakultativ vorhanden ist.

14. Expressionsvektor, umfassend ein rekombinantes DNA-Molekül nach einem der Ansprüche 10 bis 13 in operativer Verknüpfung.

15. Wirtszelle, transformiert mit einem Expressionsvektor nach Anspruch 14.

16. Wirtszelle nach Anspruch 15, **dadurch gekennzeichnet, dass** sie eine Insektenzelle, vorzugsweise eine Drosophila Schneider 2 (S2) Zelle ist.

17. Verfahren zur Herstellung eines Rubella Virus E1-E2-Hüllprotein-Komplex-Konstrukts (Synonym: Rubella-Spike-Konstrukt) gemäß Anspruch 1, umfassend die Schritte:
(a) Kultivierung von Wirtszellen, vorzugsweise Drosophila Schneider 2 (S2) Zellen;
(b) Transformieren der Wirtszellen mit einem Expressionsvektor, der eine für das Rubella E1-E2-Fusionsprotein kodierende Nukleotidsequenz in operativer Verknüpfung umfasst;
(c) Kultivierung der transfizierten Wirtszellen, wobei diese das Rubella E1-E2-Fusionsprotein exprimieren und das Rubella-Spike-Konstrukte aus der Wirtszelle sekretieren/sezerniere;
(d) Aufreinigung des Fusionsproteins.

18. Verwendung eines Rubella Virus E1-E2-Hüllprotein-Komplex-Konstrukt (Synonym: Rubella-Spike-Konstrukt) gemäß Anspruch 1 in einem Verfahren zur qualitativen und/oder quantitativen Detektion von Anti-Rubella-Antikörpern in einer Flüssigkeitsprobe als Fängerreagenz und/oder Bindungspartner für die Anti-Rubella-Antikörper.

19. Reagenzkit (Testkit) zur Durchführung eines Verfahrens zur qualitativen und/oder quantitativen Detektion von Anti-Rubella-Antikörpern in einer Flüssigkeitsprobe, wobei dieser Kit mindestens ein Rubella Virus E1-E2-Hüllprotein-Komplex-Konstrukt (Synonym: Rubella-Spike-Konstrukt) gemäß Anspruch 1 als Antigen enthält.

20. Vakzinezubereitung, die ein Rubella Virus E1-E2-Hüllprotein-Komplex-Konstrukt (Synonym: Rubella-Spike-Konstrukt) nach Anspruch 1 als antigenen Wirkstoff umfasst.

## Claims

1. Rubella virus antigen, **characterized in that** it is a rubella virus E1-E2 envelope protein complex construct (synonym: rubella spike construct) comprising
- at least one E1 component and one E2 component which are linked,
- wherein the E1 component consists of the E1 envelope protein whose C-terminal transmembrane region and intravirional domain are removed and whose N-terminus comprises the ectodomain of the E1 envelope protein,
- and wherein the E2 component consists of the E2 envelope protein whose transmembrane regions and intravirional domain are removed and whose N-terminus comprises the ectodomain of the E2 envelope protein,
- and wherein the C-terminus of the E2 component is connected to the N-terminus of the E1 component directly or by means of linkers.

2. The rubella virus antigen according to claim 1, **characterized in that** the antigen is produced as a recombinant E1-E2 fusion protein.

3. The rubella virus antigen of claim 2, **characterized in that** the E1-E2 fusion protein is coupled to a signal sequence.

4. The rubella virus antigen according to any one of claims 1 to 3, **characterized in that** the E1-E2 fusion protein is coupled to an affinity tag, preferably a streptavidin affinity tag, preferably arranged C-terminal.

5. The rubella virus antigen according to any one of claims 1 to 4, **characterized in that** the ectodomain of the E1 envelope protein comprises the amino acids (of positions) 1-446 according to SEQ ID NO:6 or the amino acids AA583-1028 of the reference sequence UniProtKB/SwissProt - P08563 (POLS_RUBVM), Sequence Update May 30, 2006 (version 2 of the sequence).

6. Rubella virus antigen according to any one of claims 1 to 5, **characterized in that** the ectodomain of the E2 envelope protein comprises the amino acids (of positions) 1-234 according to SEQ ID NO:8 or the amino acids AA301-534 of the reference sequence UniProtKB/SwissProt - P08563 (POLS RUBVM), Sequence Update May 30, 2006 (version 2 of the sequence).

7. The rubella virus antigen according to any of claims 1 to 6, **characterized in that** the linker is a flexible linker comprising glycine and/or serine.

8. Rubella virus antigen according to any one of claims 2 to 7, **characterized in that** the fusion protein comprises the amino acid sequence according to SEQ ID NO: 2, wherein the signal sequence at the N-terminal end and/or the linker sequence in the middle region of this amino acid sequence is optionally present.

9. Rubella virus antigen according to any one of claims 2 to 7, **characterized in that** the fusion protein comprises the amino acid sequence according to SEQ ID NO: 4, wherein the signal sequence at the N-terminal end and/or the linker sequence in the middle region of this amino acid sequence is optionally present.

10. A recombinant DNA molecule encoding a rubella virus antigen and comprising a nucleotide sequence encoding a rubella E1-E2 fusion protein according to claim 2.

11. Recombinant DNA molecule according to claim 10, **characterized in that** it comprises the nucleotide sequences according to SEQ ID NO: 5 and SEQ ID NO: 7.

12. Recombinant DNA molecule according to claim 10 or 11, **characterized in that** it comprises the nucleotide sequence according to SEQ ID NO: 1, wherein the signal sequence at the N-terminal end and/or the linker sequence in the middle region of this nucleotide sequence is optionally present.

13. Recombinant DNA molecule according to claim 10 or 11, **characterized in that** it comprises the nucleotide sequence according to SEQ ID NO: 3, wherein the signal sequence at the N-terminal end and/or the linker sequence in the middle region of this nucleotide sequence is optionally present.

14. An expression vector comprising a recombinant DNA molecule according to any one of claims 10 to 13 in operative linkage.

15. Host cell transformed with an expression vector according to claim 14.

16. The host cell of claim 15, **characterized in that** it is an insect cell, preferably a Drosophila Schneider 2 (S2) cell.

17. A process for preparing a rubella virus E1-E2 envelope protein complex construct (synonym: rubella spike construct) according to claim 1, comprising the steps:
(a) Cultivation of host cells, preferably Drosophila Schneider 2 (S2)cells;
(b) Transforming the host cells with an expression vector comprising a nucleotide sequence encoding the rubella E1-E2 fusion protein in operative linkage;
(c) Cultivation of the transfected host cells, whereby they express the Rubella E1-E2 fusion protein and secrete the Rubella spike constructs from the host cell;
(d) Purification of the fusion protein.

18. Use of a rubella virus E1-E2 envelope protein complex construct (synonym: rubella spike construct) according to claim 1 in a method for the qualitative and/or quantitative detection of anti-rubella antibodies in a liquid sample, as capture reagent and/or binding partner for the anti-rubella antibodies.

19. Reagent kit (test kit) for carrying out a method for the qualitative and/or quantitative detection of anti-rubella antibodies in a liquid sample, wherein this kit contains at least one rubella virus E1-E2 envelope protein complex construct (synonym: rubella spike construct) according to claim 1 as antigen.

20. A vaccine preparation comprising a rubella virus E1-E2 envelope protein complex construct (synonym: rubella spike construct) according to claim 1 as the antigenic active ingredient.

## Revendications

1. Antigène de virus de la rubéole, **caractérisé en ce qu'**il s'agit d'une construction de complexe de protéine d'enveloppe E1-E2 du virus de la rubéole (synonyme : construction de spicule de la rubéole) comprenant
- au moins un composant E1 et un composant E2 qui sont reliés,
- le composant E1 étant constitué de la protéine d'enveloppe E1 dont la région transmembranaire C-terminale et le domaine intravirional sont supprimés, et dont l'extrémité N-terminale comprend l'ectodomaine de la protéine d'enveloppe E1,
- et le composant E2 étant constitué de la protéine d'enveloppe E2 dont les régions transmembranaires et le domaine intravirional sont supprimés, et dont l'extrémité N-terminale comprend l'ectodomaine de la protéine d'enveloppe E2,
- et l'extrémité C-terminale du composant E2 étant reliée à l'extrémité N-terminale du composant E1 directement ou au moyen d'un lieur.

2. Antigène du virus de la rubéole selon la revendication 1, **caractérisé en ce que** l'antigène est produit sous forme d'une protéine de fusion E1-E2 recombinante.

3. Antigène du virus de la rubéole selon la revendication 2, **caractérisé en ce que** la protéine de fusion E1-E2 est couplée à un peptide signal.

4. Antigène du virus de la rubéole selon l'une des revendications 1 à 3, **caractérisé en ce que** la protéine de fusion E1-E2 est couplée à une étiquette, de préférence une étiquette de la streptavidine, qui est de préférence disposée en position C terminale.

5. Antigène du virus de la rubéole selon l'une des revendications 1 à 4, **caractérisé en ce que** l'ectodomaine de la protéine d'enveloppe E1 comprend les acides aminés (des positions) 1-446 selon la SEQ ID NO:6 ou les acides aminés AA583-1028 de la séquence de référence UniProtKB/SwissProt - P08563 (POLS_RUBVM), séquence mise à jour le 30 mai 2006 (version 2 de la séquence).

6. Antigène du virus de la rubéole selon l'une des revendications 1 à 5, **caractérisé en ce que** l'ectodomaine de la protéine d'enveloppe E2 comprend les acides aminés (des positions) 1-234 selon SEQ ID NO:8 ou les acides aminés AA301-534 de la séquence de référence UniProtKB/SwissProt - P08563 (POLS_RUBVM), séquence mise à jour le 30 mai 2006 (version 2 de la séquence).

7. Antigène du virus de la rubéole selon l'une des revendications 1 à 6, **caractérisé en ce que** le lieur est un lieur flexible comprenant de la glycine et/ou de la sérine.

8. Antigène du virus de la rubéole selon l'une des revendications 2 à 7, **caractérisé en ce que** la protéine de fusion comprend la séquence d'acides aminés selon SEQ ID NO:2, le peptide signal étant facultativement présent à l'extrémité N-terminale et/ou la séquence du lieur étant facultativement présente dans la région centrale de cette séquence d'acides aminés.

9. Antigène du virus de la rubéole selon l'une des revendications 2 à 7, **caractérisé en ce que** la protéine de fusion comprend la séquence d'acides aminés selon SEQ ID NO:4, le peptide signal étant facultativement présent à l'extrémité N-terminale et/ou la séquence du lieur étant facultativement présente dans la région centrale de cette séquence d'acides aminés.

10. Molécule d'ADN recombinant codant pour un antigène du virus de la rubéole et comprenant une séquence nucléotidique codant pour une protéine de fusion E1-E2 de la rubéole selon la revendication 2.

11. Molécule d'ADN recombinant selon la revendication 10, **caractérisée en ce qu'**elle comprend les séquences nucléotidiques selon SEQ ID NO:5 et SEQ ID NO:7.

12. Molécule d'ADN recombinant selon la revendication 10 ou 11, **caractérisée en ce qu'**elle comprend la séquence nucléotidique selon SEQ ID NO:1, le peptide signal étant facultativement présent à l'extrémité N-terminale et/ou la séquence du lieur étant facultativement présente dans la région centrale de cette séquence nucléotidique.

13. Molécule d'ADN recombinant selon la revendication 10 ou 11, **caractérisée en ce qu'**elle comprend la séquence nucléotidique selon SEQ ID NO:3, le peptide signal étant facultativement présent à l'extrémité N-terminale et/ou la séquence du lieur étant facultativement présente dans la région centrale de cette séquence nucléotidique.

14. Vecteur d'expression comprenant une molécule d'ADN recombinant selon l'une des revendications 10 à 13 en liaison opérationnelle.

15. Cellule hôte transformée avec un vecteur d'expression selon la revendication 14.

16. Cellule hôte selon la revendication 15, **caractérisée en ce qu'**elle est une cellule d'insecte, de préférence une cellule Schneider 2 (S2) de drosophile.

17. Procédé de production d'une construction de complexe de protéine d'enveloppe E1-E2 du virus de la rubéole (synonyme : construction de spicule de la rubéole) selon la revendication 1, comprenant les étapes suivantes :
(a) la culture de cellules hôtes, de préférence de cellules Schneider 2 (S2) de drosophile ;
(b) la transformation des cellules hôtes avec un vecteur d'expression comprenant une séquence nucléotidique codant pour la protéine de fusion E1-E2 de de la rubéole en liaison opérationnelle ;
(c) mise en culture des cellules hôtes transfectées, celles-ci exprimant la protéine de fusion E1-E2 de la rubéole, et sécrétant les constructions de spicule de la rubéole hors de la cellule hôte
(d) purification de la protéine de fusion.

18. Utilisation d'une construction de complexe de protéine d'enveloppe E1-E2 du virus de la rubéole (synonyme : construction de spicule de la rubéole) selon la revendication 1 dans un procédé de détection qualitative et/ou quantitative d'anticorps anti-rubéole dans un échantillon de liquide en tant que réactif de capture et/ou partenaire de liaison pour les anticorps anti-rubéole.

19. Kit de réactifs (kit de test) pour la mise en oeuvre d'un procédé de détection qualitative et/ou quantitative d'anticorps anti-rubéole dans un échantillon de liquide, ledit kit contenant au moins une construction de complexe de protéine d'enveloppe E1-E2 du virus de la rubéole (synonyme : construction de spicule de la rubéole) selon la revendication 1 en tant qu'antigène.

20. Préparation d'un vaccin comprenant une construction de complexe de protéine d'enveloppe E1-E2 du virus de la rubéole (synonyme : construction de spicule de la rubéole) selon la revendication 1 en tant que principe actif antigénique.
